# EUROPEAN PATENT APPLICATION

(11) **EP 4 063 489 A1**
(43) Date of publication of application: **28.09.2022**
(21) Application number: 20899161.2
(22) Date of filing: 11.12.2020
(51) Int. Cl.: C12N 5/078, A61K 35/19, A61K 38/18, A61P 17/00, A61P 17/02, A61P 17/14, A61P 19/02, A61P 19/04, A61P 43/00, C12N 5/0775

(54) **COMPOSITION AND USE THEREOF**

(30) Priority: 13.12.2019 JP 2019225959
(71) Applicant: Megakaryon Corporation, Kyoto-shi, Kyoto 6008813 (JP)
(72) Inventor: HAZAMA Yasuko, Kyoto-shi, Kyoto 600-8813 (JP); INOUE Tomoyuki, Kyoto-shi, Kyoto 600-8813 (JP); SATAKE Makoto, Kyoto-shi, Kyoto 600-8813 (JP); SHIGEMORI Tomohiro, Kyoto-shi, Kyoto 600-8813 (JP)
(74) Representative: Schön, Christoph
(86) International application number: PCT/JP2020/046426
(87) International publication number: WO 2021/117900

(57) **Abstract**

A composition having cell-derived physiological activity is provided. The composition according to the present invention contains a treated product of megakaryocytes or a culture of the megakaryocytes.

SEQUENCE LISTING

<110>  MEGAKARYON CORPORATION

<120>  Composition and use thereof

<130>  TF19291WO

<150>  JP2019-225959
<151>  2019-12-13

<160>  10

<170>  PatentIn version 3.5

<210>  1
<211>  25
<212>  DNA
<213>  Artificial Sequence

<220>
<223>  Forward primer for GAPDH gene

<400>  1
catccctgcc tctactggcg ctgcc                                          25


<210>  2
<211>  25
<212>  DNA
<213>  Artificial Sequence

<220>
<223>  Reverse Primer for GAPDH gene

<400>  2
ccaggatgcc cttgaggggg ccctc                                          25


<210>  3
<211>  24
<212>  DNA
<213>  Artificial Sequence

<220>
<223>  Forward Primer for FLG gene

<400>  3
tcggcaaatc ctgaagaatc caga                                           24


<210>  4
<211>  26
<212>  DNA
<213>  Artificial Sequence

<220>
<223>  Reverse Primer for FLG gene

<400>  4
gcttgagcca acttgaatac catcag                                         26

<210>  5
<211>  30
<212>  DNA
<213>  Artificial Sequence

<220>
<223>  Forward Primer for SPTLC1 gene

<400>  5
acaaagcaag aatcttcctg gaggaaagcc                                        30


<210>  6
<211>  30
<212>  DNA
<213>  Artificial Sequence

<220>
<223>  Reverse Primer for SPTLC1 gene

<400>  6
aaacctccaa tagaagcaag tgcattctcc                                        30


<210>  7
<211>  25
<212>  DNA
<213>  Artificial Sequence

<220>
<223>  Forward Primer for FGF7 gene

<400>  7
tctgtcgaac acagtggtac ctgag                                             25


<210>  8
<211>  22
<212>  DNA
<213>  Artificial Sequence

<220>
<223>  Reverse Primer for FGF7 gene

<400>  8
gccactgtcc tgatttccat ga                                                22


<210>  9
<211>  25
<212>  DNA
<213>  Artificial Sequence

<220>
<223>  Forward Primer for VEGFA gene

<400>  9
aaagcatttg tttgtacaag atccg                                             25


<210>  10
<211>  25
<212>  DNA

<213>  Artificial Sequence

<220>
<223>  Reverse Primer for VEGFA gene

<400>  10
cttgtcacat ctgcaagtac gttcg                                          25

## Description

### TECHNICAL FIELD

The present invention relates to a composition and use thereof.

### BACKGROUND ART

Attempts have been made to develop, cells that facilitate tissue regeneration, such as mesenchymal stem cells and the like, as cell preparations serving as therapeutic drugs for regenerative medicine. Further, it is conceivable that cells that facilitate the tissue regeneration facilitate tissue regeneration by releasing proteins having physiological activities, such as growth factors and the like.

### SUMMARY OF INVENTION

### Technical Problem

In view of this, the present invention aims to provide a composition having cell-derived physiological activity.

### Solution to Problem

In order to achieve the aim, a composition according to the present invention contains a treated product of megakaryocytes or a culture of the same.

A cell proliferation promoting composition (hereinafter also referred to as "proliferation promoting composition") according to the present invention contains the composition of the present invention.

A fibroblast function promoting composition according to the present invention contains the composition of the present invention.

A composition for promoting healing of a skin disorder according to the present invention contains the composition of the present invention.

A keratinocyte function promoting composition according to the present invention contains the composition of the present invention.

A dermal papilla cell function promoting composition according to the present invention contains the composition of the present invention.

A hair growth promoting composition according to the present invention contains the composition of the present invention.

### Advantageous Effects of Invention

According to the present invention, it is possible to provide a composition having cell-derived physiological activity.

### BRIEF DESCRIPTION OF DRAWINGS

[FIG. 1] FIG. 1 is a schematic diagram showing a configuration of a concentrating system in Example 1.
[FIG. 2] FIG. 2 is graphs showing proliferative activity of cells in Example 1, in which FIG. 2(A) shows relative values of the proliferative activity and FIG. 2(B) shows the doubling time.
[FIG. 3] FIG. 3 is graphs showing the proliferative activity of cells in Example 2, in which FIG. 3(A) shows relative values of the proliferative activity and FIG. 3(B) shows the doubling time.
[FIG. 4] FIG. 4 is a graph showing the proliferative activity of fibroblasts in Example 3.
[FIG. 5] FIG. 5 is a graph showing the amount of Type I collagen produced in Example 3.
[FIG. 6] FIG. 6 is a graph showing the amount of hyaluronic acid produced in Example 3.
[FIG. 7] FIG. 7 is photographs showing phase-contrast images of cells in Example 4.
[FIG. 8] FIG. 8 is a graph showing the proliferative activity of keratinocytes in Example 4.
[FIG. 9] FIG. 9 is a graph showing the expression level of the FLG gene in Example 4.
[FIG. 10] FIG. 10 is a graph showing the expression level of the SPTLC1 gene in Example 4.
[FIG. 11] FIG. 11 is a graph showing the proliferative activity of dermal papilla cells in Example 5.
[FIG. 12] FIG. 12 is a graph showing the expression level of the FGF7 gene in Example 5.
[FIG. 13] FIG. 13 is a graph showing the expression level of the VEGFA gene in Example 5.

### DESCRIPTION OF EMBODIMENTS

### Composition

As described above, a composition according to the present invention contains a treated product of megakaryocytes or a culture thereof. As described above, a composition according to the present invention contains a treated product of megakaryocytes or a culture thereof, and there is no limitation on other constituents or conditions. According to the composition of the present invention, for example, it is possible to provide a composition having cell-derived physiological activity. It is expected that it is possible to suitably use the composition of the present invention, for example, for promoting proliferation of cells such as mesenchymal cells, fibroblasts, keratinocytes, and dermal papilla cells, for promoting healing of skin disorders such as skin ulcers, pressure sores, burns, scars, wounds, and skin aging, for maintaining or improving the barrier function of skin, and for hair growth, and the like.

In the present invention, a "megakaryocyte" refers to the largest cell present in bone marrow in an organism, and refers to a cell that releases platelets and a cell having an equivalent function. The cell having the equivalent function refers to a cell having an ability to produce platelets. In the present invention, megakaryocytes may be pre-multinuclear (polyploid) megakaryocytes, that is, immature megakaryocytes or proliferative megakaryocytes, or post-multinuclear megakaryocytes (multinucleated megakaryocytes). As a specific example, the megakaryocyte may be a promegakaryoblast, a megakaryoblast, a promegakaryocyte, or a mature megakaryocyte. The number of sets of chromosomes in the multinucleated megakaryocyte need only exceed two sets, and specific examples thereof include from 16 to 32 sets.

There is no particular limitation on the origin of the megakaryocytes, and examples thereof include humans and non-human animals. Examples of the non-human animals include primates, such as monkeys, gorillas, chimpanzees, and marmosets, and mice, rats, dogs, cats, rabbits, sheep, horses, and guinea pigs. Hereinafter, the same applies to the origin of the other cells.

In the present invention, the megakaryocytes can be identified by cell surface markers. If the megakaryocytes are derived from a human, examples of the cell surface marker include CD41a, CD42a, and CD42b. That is, the megakaryocytes are cells positive for CD41a, CD42a, and CD42b. If the megakaryocytes are derived from a human, the cell surface marker may be, for example, at least one selected from the group consisting of CD9, CD61, CD62p, CD42c, CD42d, CD49f, CD51, CD110, CD123, CD131, and CD203c.

The megakaryocytes may be megakaryocytes isolated from an organism, or may also be megakaryocytes derived from cells that are less differentiated than megakaryocytes, such as pluripotent cells (hereinafter also referred to as "progenitors"). The "cells that are less differentiated than megakaryocytes" refer to cells having an ability to differentiate into the megakaryocytes.

If the megakaryocytes are megakaryocytes isolated from an organism, the megakaryocytes are present in bone marrow, and thus can be isolated, for example, from the bone marrow. In this case, the megakaryocytes may contain other cells derived from the organism.

If the megakaryocytes are megakaryocytes derived from progenitors, as will be described later, the megakaryocytes can be induced *in vitro.* In this case, the megakaryocytes may contain the progenitors. Examples of the progenitors include hematopoietic stem cells, hematopoietic progenitor cells, CD34-positive cells, megakaryocyte-erythroid progenitors (MEPs), and megakaryocyte progenitors. The progenitor cell may be isolated from bone marrow, umbilical cord blood, peripheral blood, or the like, or may be derived from pluripotent cells such as ES cells (embryonic stem cells), induced pluripotent stem cells (iPS cells), nuclear transfer ES cells (ntES cells), reproductive stem cells, somatic stem cells, embryonic tumor cells, or the like.

If the megakaryocytes are megakaryocytes derived from progenitor cells, the megakaryocytes are preferably immortalized megakaryocytes. The immortalized megakaryocytes have higher homogeneity in the cell differentiation stage than megakaryocytes induced using other megakaryocyte induction methods, for example, and thus it is possible to suppress a change in the component composition in the resulting treated product. For example, as will be described later, the immortalized megakaryocytes are megakaryocytes induced by introducing an oncogene and a Polycomb gene, or an oncogene, a Polycomb gene, and an apoptosis suppressor gene, into the progenitors.

The "oncogene" refers to a gene that is capable of inducing canceration of cells in an organism, and examples thereof include MYC family genes, such as c-MYC, N-MYC, and L-MYC, and the like, SRC family genes, RAS family genes, RAF family genes, c-kit (CD117), PDGFRs (platelet-derived growth factor receptors), and protein kinase family genes, such as Abl (Abelson murine leukemia viral oncogene homolog) and the like.

The "Polycomb gene" refers to a gene that are known to function for negatively regulating CDKN2a (cyclin-dependent kinase inhibitor 2A, INK4a/ARF) and avoiding cellular aging (References 1 to 3 below). Specific examples of the Polycomb gene include BMI1 (Polycomb complex protein BMI-1, Polycomb group RING finger protein 4 (PCGF4), and RING finger protein 51 (RNF51)), Mel18 (Polycomb group RING finger protein 2), Ring (Ring Finger Protein) 1a/b, Phc (Polyhomeotic Homolog) 1/2/3, Cbx (Chromobox) 2/4/6/7/8, Ezh2 (Enhancer Of Zeste 2 Polycomb Repressive Complex 2 Subunit), Eed (Embryonic Ectoderm Development), Suz12 (SUZ12 Polycomb Repressive Complex 2 Subunit), HADC (Histone deacetylases), and Dnmt (DNA (cytosine-5)-methyltransferase)1/3a/3b, and the like. Reference 1: Hideyuki Oguro et al., "Senescence and Aging of Stem Cells Regulated by Polycomb Complexes", Regenerative Medicine, 2007, vol. 6, no. 4, pages 26 - 32 Reference 2: Jesus Gil et al., "Regulation of the INK4b-ARF-INK4a tumour suppressor locus: all for one or one for all", Nature Reviews Molecular Cell Biology, 2007, vol. 7, pages 667-677 Reference 3: Soo-Hyun Kim et al., "Absence of p16INK4a and truncation of ARF tumor suppressors in chickens", PNAS, 2003, vol. 100, No.1, pages 211-216

The "apoptosis suppressor gene" refers to a gene that functions to be capable of suppressing cell apoptosis, and examples thereof include BCL2 (B-cell lymphoma 2), Bcl-xL (B-cell lymphoma-extra large), Survivin (Baculoviral IAP Repeat Containing 5), and MCL1 (BCL2 Family Apoptosis Regulator), and the like.

The immortalized megakaryocytes are preferably megakaryocytes that contain exogenous BMI1 gene, MYC gene, and Bcl-xL gene. The "exogenous" refers to being introduced into a cell from outside the cell. The "exogenous gene" may be present on a chromosome of the cell or in the nucleus or cytoplasm. The exogenous gene can be detected, for example, by measuring a number of these genes. If the gene is a gene on an autosome, one gene is present on each autosome, and thus, one cell has two genes. Thus, in the absence of the exogenous gene, two of the genes are detected in one cell. On the other hand, in the presence of the exogenous gene, three or more of the genes are detected in one cell. In this case, the exogenous gene can be detected, for example, using PCR with use of a primer, a probe, or a combination thereof, or the like. If the exogenous gene has a tag sequence or a selection marker, the exogenous gene may be detected by detecting the tag sequence or the selection marker. Also, the exogenous gene can be detected, for example, using an antibody against a protein translated from the gene, or the like.

A culture of the megakaryocytes is a culture produced, for example, by culturing the megakaryocytes. For example, as will be described later, the megakaryocytes are cultured by culturing the megakaryocytes in the presence of a culture medium.

The culture of the megakaryocytes can be obtained by culturing the megakaryocytes, and thus the culture of the megakaryocytes is a mixture containing, as cell components, the megakaryocytes and platelets produced from the megakaryocytes. The cell components refer to cells and platelets. As described above, the megakaryocytes can be derived from cells that are less differentiated than the megakaryocytes. Thus, if megakaryocytes that are derived from the less differentiated cells than the megakaryocytes are contained as megakaryocytes for producing the culture of the megakaryocytes, the culture of the megakaryocytes may contain the less differentiated cells than the megakaryocytes.

In the present invention, the culture of the megakaryocytes may be a culture obtained by culturing the megakaryocytes or a culture obtained by processing the culture. The culture may be processed, for example, through removal of a liquid fraction, extraction of a cell component fraction, changing of a composition of the cell components that include platelets, or the like. The composition of the cell components can be changed, for example, through removal of cells and/or platelets from the mixture, extraction of cells and/or platelets from the mixture, addition of cells and/or platelets to the mixture, or the like.

The "platelets" are one type of the cell components in blood, and refer to a cell component that is positive for CD41a and CD42b. The platelets, for example, do not have a cell nucleus, and the size of the platelets is smaller than that of the megakaryocytes. Thus, the platelets and the megakaryocytes can be distinguished, for example, by the presence/absence of cell nuclei and/or the size. It is known that the platelets play an important role in thrombus formation and hemostasis, and are also involved in the pathophysiology of tissue regeneration and inflammation after injury. Further, it is known that, if platelets are activated due to bleeding or the like, receptors for cell adhesion factors, such as Integrin αIIBβ3 (glycoprotein IIb/IIIa; a complex of CD41a and CD61) or the like, are expressed on the platelet membrane. Also, if the platelets are activated, the platelets aggregate, and fibrin coagulates due to various blood coagulation factors released from the platelets. Therefore, a thrombus is formed, and hemostasis progresses. In the present invention, the origin of the platelets is the same as the origin of the megakaryocytes.

In the present invention, the treated product may be prepared from the megakaryocytes, or may be prepared from the culture of the megakaryocytes. Further, if the treated product is prepared from the culture of the megakaryocytes, the culture of the megakaryocytes may be processed. As a specific example, the treated product may be a product obtained by treating a cell fraction or a liquid fraction of the megakaryocytes or the culture thereof, or may be a product obtained by treating a processed product of the megakaryocytes or the culture thereof. There is no particular limitation on treatment in the preparation of the treated product, and examples of the treatment include treatments for changing a density of cell components, such as a concentrating treatment, a separation treatment, or a purification treatment, or the like; extraction treatments for extracting cell components, such as a drying treatment, a freezing treatment, a freeze-drying treatment, a solvent treatment, a surfactant treatment, an enzyme treatment, or a protein fraction extraction treatment or the like; and disruption treatments, such as a homogenization treatment, or a pulverization treatment, or the like. Specific examples of the treated product include extracts of concentrates, dried products, frozen products, freeze-dried products, solvent-treated products, surfactant-treated products, enzyme-treated products, protein fractions, or sonicated products, or the like, of the megakaryocytes or the culture thereof; disrupted products, such as homogenized products and pulverized products; extracts of concentrates, dried products, frozen products, freeze-dried products, solvent-treated products, surfactant-treated products, enzyme-treated products, protein fractions, or sonicated products, or the like, of a cell fraction of the megakaryocytes or the culture thereof; and disrupted products, such as homogenized products and pulverized products, and the like. The treated product may be made of one type of treated product or may be a mixture of two or more types of treated products. There is no particular limitation on the mixture, and can be a mixture obtained by mixing treated products in any combination and ratio.

The treated product contains, for example, at least one of one or more growth factors or growth factor receptors. Also, the treated product, for example, has physiological activity such as cell proliferation promoting activity. Further, as described above, the treated product can be produced, for example, by treating the megakaryocytes or the culture thereof. Thus, in the present invention, the treated product can be specified, for example, using the conditions (1) to (3) below. The treated product may be specified, for example, using any one of the conditions (1) to (3) below, may be specified using multiple conditions, or may be specified by all of the conditions. As a specific example, the treated product can be specified, for example, using a combination of the conditions.

### (Conditions)

(1) Content of growth factors and/or growth factor receptors;
(2) Physiological activity of a treated product;
(3) Method for producing a treated product

### (Combination of conditions)

Condition (1), (2), or (3);
Conditions (1) and (2), Conditions (1) and (3), or Conditions (2) and (3);
Conditions (1), (2), and (3)

### (1) Condition (1)

As described above, the condition (1) is a condition regarding to the content of the growth factor and/or the growth factor receptor. In the above condition (1), the content of the growth factor or the content of the growth factor receptor may be specified, or the content of the growth factor and the content of the growth factor receptor may be specified. Also, one type or two or more types of growth factors may be specified in the condition (1). One type or two or more types of growth factor receptors may be specified in the condition (1).

In the condition (1), examples of the growth factor include basic fibroblast growth factor (bFGF), insulin-like growth factor-binding protein-1 (IGFBP-1), insulin-like growth factor-binding protein-2 (IGFBP-2), insulin-like growth factor-binding protein-3 (IGFBP-3), insulin-like growth factor-binding protein-6 (IGFBP-6), placental growth factor (PIGF), vascular endothelial growth factor (VEGF), endocrine gland-derived vascular endothelial growth factor (EG-VEGF), growth differentiation factor-15 (GDF-15), amphiregulin (AR), bone morphogenetic protein-5 (BMP-5), bone morphogenetic protein-7 (BMP-7), hepatocyte growth factor (HGF), and TGFβ1 (transforming growth factor β1), and the like.

In the condition (1), examples of the growth factor receptor include stem cell factor receptor (SCFR), epidermal growth factor receptor (EGFR), and vascular endothelial growth factor receptor 2 (VEGFR2), and the like.

The content may be, for example, weight of the growth factors and the growth factor receptors in the treated product, or weight of the growth factors and the growth factor receptors relative to weight of a total protein in the treated product (a content in the total protein). However, the latter is preferable.

The weight of the total protein can be determined, for example, using the BCA protein assay. The BCA protein assay is a protein assay in which coordinate bonding between a monovalent copper ion and two molecules of bicinchoninic acid is used. It is preferable that samples to be subjected to the BCA protein assay do not contain, for example, a reducing agent and/or a chelating agent for copper ions. The BCA protein assay can be carried out according to Reference 4 below, and for example, a commercially available kit or the like may be used. Pierce^{™} BCA Protein Assay Kit (manufactured by Thermo Fisher Scientific) or the like can be used as a kit for the BCA protein assay.

Reference 4: Toshiharu Hase et al., "Protein Science Experimental Method 1 based on Easy Principles, Production of Protein, Extraction/Purification and Synthesis", Kagaku-Dojin, December 13, 2008

The total protein concentration in the treated product can be set as appropriate, for example, by a number of cells to be subjected to treatment and a volume of a solvent in the treated product. The total protein concentration in the treated product can be made comparatively high, for example, by increasing the number of cells to be subjected to the treatment or reducing the volume in the solvent in the treated product, or through extraction from the megakaryocytes. Also, the total protein concentration in the treated product can be made comparatively low, for example, by reducing the number of cells to be subjected to the treatment or increasing the volume of the solvent in the treated product, or through extraction from the culture of the megakaryocytes. As a specific example, if the number of cells to be subjected to the treatment is 1×10⁸ cells and the volume of the solvent in the treated product is 100 µl, the total protein concentration in the treated product is, for example, 0.1 to 200 mg/ml. The solvent is, for example, an aqueous solvent, which will be described later.

The weight of the growth factor and the weight of the growth factor receptor can be determined, for example, through a sandwich ELISA. The sandwich ELISA can be carried out according to Reference 5 below, and, for example, a commercially available kit or the like may be used. Quantibody ^{®} Human Growth Factor Array 1 (manufactured by RayBiotech Inc.) or the like can be used as a kit for the sandwich ELISA.

Reference 5: "Immunoassay, from Basic to Advanced" edited by Biochemical Assay Society of Japan, Kodansha, December 20, 2014.

Examples of the content of the growth factor and the content of the growth factor receptor in the treated product include the followings.

If the growth factor is bFGF, the treated product contains bFGF, for example in an amount of 2000 to 20000 pg, 5000 to 20000 pg, or 10000 to 20000 pg, in 1 mg of the total protein. As will be described later, for example, the treated product contains bFGF, and thus the treated product exhibits cell proliferation promoting activity.

If the growth factor is IGFBP-1, the treated product contains IGFBP-1 in an amount of 0 to 200 pg, 0.01 to 200 pg, 0.01 to 100 pg, or 0.01 to 50 pg, in 1 mg of the total protein.

If the growth factor is IGFBP-2, the treated product contains IGFBP-2 in an amount of 8000 to 80000 pg, 10000 to 80000 pg, or 20000 to 80000 pg, in 1 mg of the total protein.

If the growth factor is PIGF, the treated product contains PIGF in an amount of 1 to 60 pg, 1 to 30 pg, or 1 to 20 pg, in 1 mg of the total protein.

If the growth factor is VEGF, the treated product contains VEGF in an amount of 20 to 800 pg, 20 to 600 pg, or 20 to 400 pg, in 1 mg of the total protein.

If the growth factor is GDF-15, the treated product contains GDF-15 in an amount of 1000 to 10000 pg, 1000 to 5000 pg, or 2000 to 5000 pg, in 1 mg of total protein.

If the growth factor is AR, the treated product contains AR in an amount of 0 to 16 pg, 0.01 to 16 pg, 0.1 to 16 pg, or 1 to 16 pg in 1 mg of the total protein.

If the growth factor is HGF, the treated product contains HGF in an amount of 0 to 100 pg, 0.01 to 100 pg, 0.01 to 50 pg, or 0.01 to 30 pg in 1 mg of the total protein.

If the growth factor is BMP-7, the treated product contains BMP-7 in an amount of 0 to 1000 pg or 0.01 to 1000 pg, in 1 mg of the total protein.

If the growth factor receptor is SCFR, the treated product contains SCFR in an amount of 200 to 2000 pg, 300 to 1500 pg, or 400 to 1000 pg, in 1 mg of total protein.

If the growth factor receptor is EGFR, the treated product contains EGFR in an amount of 0 to 60 pg, 0.01 to 60 pg, 1 to 50 pg, 1 to 45 pg, or 10 to 40 pg, in 1 mg of the total protein.

If the growth factor receptor is VEGFR2, the treated product contains VEGFR2 in an amount of 20 to 400 pg, 50 to 350 pg, or 100 to 300 pg, in 1 mg of the total protein.

As described above, the condition (1) may be specified by the content of one or two or more growth factors, or may be specified by the content of one or two or more growth factor receptors, or may be specified by any combination of these contents. In this case, the condition (1) is specified, for example, with at least 1, 2, 3, 4, 5, 6, 7, 8, 9, 10, 11, or 12 conditions selected from the group consisting of the following conditions (A1) to (A9) and (B1) to (B3). As a specific example, the following combinations can be exemplified as combinations of the content of the growth factors and/or the content of growth factor receptors.

### (Combination of content of growth factors and/or content of growth factor receptors)

Any one of conditions (A1) to (A9) and (B1) to (B3):
(A1) the bFGF content, (A2) the IGFBP-1 content, (A3) the IGFBP-2 content, (A4) the PIGF content, (A6) the VEGF content, (A6) the GDF-15 content, (A7) the AR content, (A8) the HGF content, (A9) the BMP-7 content, (B1) the SCFR content, (B2) the EGFR content, or (B3) the VEGFR2 content;
Any two of the conditions (A1) to (A9) and (B1) to (B3):
   (A1) and (A2), (A1) and (A3), (A1) and (A4), (A1) and (A5), (A1) and (A6), (A1) and (A7), (A1) and (A8), (A1) and (A9), (A1) and (B1), (A1) and (B2), (A1) and (B3),
   (A2) and (A3), (A2) and (A4), (A2) and (A5), (A2) and (A6), (A2) and (A7), (A2) and (A8), (A2) and (A9), (A2) and (B1), (A2) and (B2), (A2) and (B3),
   (A3) and (A4), (A3) and (A5), (A3) and (A6), (A3) and (A7), (A3) and (A8), (A3) and (A9), (A3) and (B1), (A3) and (B2), (A3) and (B3),
   (A4) and (A5), (A4) and (A6), (A4) and (A7), (A4) and (A8), (A4) and (A9), (A4) and (B1), (A4) and (B2), (A4) and (B3),
   (A5) and (A6), (A5) and (A7), (A5) and (A8), (A5) and (A9), (A5) and (B1), (A5) and (B2), (A5) and (B3),
   (A6) and (A7), (A6) and (A8), (A6) and (A9), (A6) and (B1), (A6) and (B2), (A6) and (B3),
   (A7) and (A8), (A7) and (A9), (A7) and (B1), (A7) and (B2), (A7) and (B3),
   (A8) and (A9), (A8) and (B1), (A8) and (B2), (A8) and (B3),
   (A9) and (B1), (A9) and (B2), (A9) and (B3),
   (B1) and (B2), (B1) and (B3), or
   (B2) and (B3).

The content in the total protein may be adjusted according to applications of the composition of the present invention, for example, by adding or removing proteins other than the growth factors and the growth factor receptors. Examples of the other proteins include proteins that do not affect the activities of the growth factor and the growth factor receptors, and specific examples thereof include serum albumin such as human serum albumin or the like, and gamma globulins such as human gamma globulin or the like, and the like. Examples of the removal of the protein include removal using a column, and removal using an antibody, and the like.

### (2) Condition (2)

As described above, the condition (2) is a condition regarding the physiological activity of a treated product. In the condition (2), the physiological activity of the treated product refers to, for example, the activity for regulating the function of cells, tissues, or organs. Examples of the function of cells include proliferation, differentiation, induction or suppression of gene expression, and induction or suppression of the expression of biopolymers such as proteins and glycans.

Examples of the physiological activity of the treated product include cell proliferation promoting activity, fibroblast function promoting activity, keratinocyte function promoting activity, and dermal papilla cell function promoting activity. In the cell proliferation promoting activity, there is no particular limitation on the cells, and examples thereof include mesenchymal stem cells, fibroblasts, keratinocytes such as epidermal keratinocytes, and dermal papilla cells such as scalp dermal papilla cells. The treated product may have, for example, one activity or multiple activities.

The mesenchymal stem cells refer to cells that have anability to self-renew and have an ability to differentiate into bone, cartilage, and fat cells. The mesenchymal stem cells can be specified using a cell surface marker. The mesenchymal stem cells are, for example, positive for CD73, CD90, and CD105, and are negative for CD14, CD34, and CD45.

The fibroblasts are cells that constitute a connective tissue of an organ such as skin, lung, or heart, and cells for supplying fiber components (extracellular matrix components such as collagen, elastin, and hyaluronic acid). The fibroblasts can be specified using a cell surface marker. The fibroblasts are, for example, positive for vimentin, CD90, and TE-7 antibody.

Among epidermal cells, the keratinocytes are cells that have a keratinization ability and divide in the basal layer of the epidermis and contribute to epidermis formation. The keratinocytes can be specified using a cell surface marker. The keratinocytes are, for example, positive for the androgen receptor and cytokeratins. The keratinocytes are preferably epidermal keratinocytes.

The dermal papilla cells are cells that are present in the dermal papilla at the base of the hair follicle and are important for inducing and maintaining hair growth. The dermal papilla cells can be specified using a cell surface marker and/or gene expression. The dermal papilla cells are, for example, positive for alkaline phosphatase; and positive for SOX2 gene, WIF 1 gene, Noggin gene, BMP4 gene, and VCAN gene. The dermal papilla cells are preferably scalp dermal papilla cells.

The cell proliferation promoting activity is, for example, applicable as long as the cell proliferation ability is promoted compared with the control group that is the same except that the composition of the present invention is not added, and, for example, the cell proliferative ability may decrease, compared to the cell proliferative ability at the beginning. In this case, the "proliferation promoting activity" may also be referred to for example, as suppressing a decrease in the proliferative activity. As a specific example, the proliferative activity of the mesenchymal stem cells decreases every time subculturing is performed. Because the composition of the present invention can suppress a decrease in the proliferative activity of the mesenchymal stem cells, it can be said, for example, that the composition of the present invention exhibits proliferative activity. The cell proliferation promoting activity can be measured, for example, under culture conditions in which the target cells proliferate. The culture conditions, for example, can be set as appropriate according to the type of the cells. As a specific example, if mesenchymal stem cells are used as the cells, the proliferative activity of the cells can be measured, for example, by culturing human-derived mesenchymal stem cells *in vitro* in a growth medium. The proliferative activity of the mesenchymal stem cells can be measured, for example, based on Example 1, which will be described later. Also, if fibroblasts are used as the cells, the proliferative activity of the cells can be measured, for example, by culturing human-derived fibroblasts *in vitro* in a growth medium. The proliferative activity of the fibroblasts can be measured, for example, based on Example 3, which will be described later. If keratinocytes are used as the cells, the proliferative activity of the cells can be measured, for example, by culturing human-derived epidermal keratinocytes *in vitro* in a growth medium. The proliferative activity of the keratinocytes can be measured, for example, based on Example 4, which will be described later. If dermal papilla cells are used as the cells, the proliferative activity of the cells can be measured, for example, by culturing human-derived hair dermal papilla cells *in vitro* in a growth medium. The proliferative activity of the dermal papilla cells can be measured, for example, based on Example 5, which will be described later.

The fibroblast function promoting activity is, for example, applicable as long as the fibroblast function is improved compared twith the control group that is the same except that the composition of the present invention is not added. The function of the fibroblast may refer to, for example, either the proliferation of the fibroblast or the production of the extracellular matrix by the fibroblast. Examples of the extracellular matrix include fibrous substances such as collagens such as Type I collagen, and elastin; and matrix-like substances, such as glycosaminoglycans such as hyaluronic acid and chondroitin sulfate, and cell-adhesive proteins such as proteoglycans, integrins, fibronectins, and laminins. The function of the fibroblasts can be measured, for example, based on Example 3, which will be described later.

The keratinocyte function promoting activity is, for example, applicable as long as the keratinocyte function is improved as compared with the control group that is the same except that the composition of the present invention is not added. The function of the keratinocyte may refer to, for example, any one of the proliferation of the keratinocyte, the differentiation into epidermal cells, or the induction of barrier function genes. The barrier function gene refers to, for example, a gene that functions to maintain the barrier function of the skin, and specific examples thereof include profilaggrin gene (FLG) and ceramide synthase gene (serine palmitoyltransferase long chain base subunit 1: SPTLC1). As the profilaggrin gene, examples of the human-derived profilaggrin gene include a polynucleotide having a base sequence registered in Genbank under Accession No.: NM_002016.2. As the ceramide synthase gene, examples of the human-derived ceramide synthase gene include a polynucleotide having a base sequence registered in Genbank under Accession No.: NM_001281303.2. The function of the keratinocytes can be measured, for example, based on Example 4, which will be described later.

The dermal papilla cell function promoting activity is, for example, applicable as long as the dermal papilla cell function is improved compared with the control group that is the same except that the composition of the present invention is not added. The function of the dermal papilla cells may refer to, for example, either the proliferation of the dermal papilla cells or the induction of hair growth promoting genes. The hair growth promoting gene refers to, for example, a gene that functions to regrow hair and to grow or maintain hair, and specific examples thereof include FGF7 (fibroblast growth factor 7) gene and vascular endothelial growth factor (VEGF) gene. Examples of the VEGF include VEGFA. As the FGF7 gene, examples of the human-derived pro-FGF7 gene include a polynucleotide having a base sequence registered in Genbank under Accession No.: NM_002009.4. As the VEGFA gene, examples of the human-derived VEGFA gene include a polynucleotide having a base sequence registered in Genbank under Accession No.: NM_001025366.3. The function of the dermal papilla cells can be measured, for example, based on Example 5, which will be described later.

### (3) Condition (3)

As described above, the condition (3) is a condition regarding a method for producing a treated product. A description of a method for producing a composition of the present invention, which will be described later, can be applied to the method for producing a treated product in the composition of the present invention.

The composition of the present invention can be produced, for example, using a method for producing the composition of the present invention, which will be described later.

As will be described later, the composition of the present invention can be used, for example, as a cell proliferation promoting composition, a fibroblast function promoting composition, a keratinocyte function promoting composition, a dermal papilla cell function promoting composition, or the like. Also, based on the above-described activities, it is expected that the composition of the present invention can be suitably used, for example, as a composition for promoting healing of a skin disorder and a hair growth promoting composition. A description of a cell proliferation promoting composition, a fibroblast function promoting composition, a keratinocyte function promoting composition, and a dermal papilla cell function promoting composition, which will be described later, can be applied to a method of use according to the present invention. Further, the composition of the present invention can be used, for example, for repairing knee joint injuries, tendon injuries, or ligament injuries; curing ulcers, pressure sores, burns, scars, or wounds; improving skin texture; hair growth; and/or skin beautification.

### Method for producing composition

A method for producing a composition of the present invention (hereinafter also referred to as "production method") includes a treating step of treating megakaryocytes or a culture thereof. The production method according to the present invention includes the treating step, and there is no particular limitation on other steps and conditions. According to the production method of the present invention, it is possible to produce the composition of the present invention. The above descriptions of the composition of the present invention can be applied to the production method of the present invention.

In the production method of the present invention, an object to be treated in the treating step is megakaryocytes or a culture thereof. Thus, the production method of the present invention may include a megakaryocyte inducing step that induces the megakaryocytes from cells that are less differentiated than the megakaryocytes and/or a producing step that produces a culture of the megakaryocytes, prior to the treating step.

In the megakaryocyte inducing step, there is no particular limitation on the method for inducing the megakaryocytes, and the megakaryocytes can be induced using a known induction method. As a specific example, with regard to the method for inducing the megakaryocytes, the method for inducing immortalized megakaryocytes disclosed in WO 2011/034073 (US 2012/0238023A), WO 2012/157586 (US 2014/0127815A), WO 2014/123242 (US 2016/0002599A), and the like; the method for inducing megakaryocytes described in Reference 6 below; and the like can be referred to, and the method for inducing megakaryocytes is incorporated herein by reference as part of this specification. As a specific example, in the megakaryocyte inducing step, for example, the oncogene and the Polycomb gene may be forcibly expressed in the cells that are less differentiated than the megakaryocytes. As a result, it is possible to obtain, for example, immortalized megakaryocytes that proliferate infinitely in the megakaryocyte inducing step. Further, for example, by halting the forced expression of the immortalized megakaryocytes, the immortalized megakaryocytes can be induced into multinucleated megakaryocytes to produce platelets. Also, in the megakaryocyte inducing step, for example, the apoptosis suppressor gene may be forcibly expressed in the megakaryocyte progenitors. As a result, it is possible to obtain the immortalized megakaryocytes in the megakaryocyte inducing step. Further, for example, by halting the forced expression of the immortalized megakaryocytes, the immortalized megakaryocytes can be induced into multinucleated megakaryocytes to produce platelets.

Reference 6: Ann-Kathrin Borger et al., "Generation of HLA-Universal iPSC-Derived Megakaryocytes and Platelets for Survival Under Refractoriness Conditions", Mol. Med., 2016, vol. 22, pages 274-288

In the megakaryocyte inducing step, for example, the oncogene, the Polycomb gene, and the apoptosis suppressor gene may also be forcibly expressed. In this case, the oncogene, the Polycomb gene, and the apoptosis suppressor gene may also be forcibly expressed, simultaneously or separately. As specific examples, in the megakaryocyte inducing step, after the oncogene and the Polycomb gene are forcibly expressed, the forced expression may be halted, and then the apoptosis suppressor gene may be forcibly expressed, or the oncogene, the Polycomb gene, and the apoptosis suppressor gene may be forcibly expressed, or the oncogene and the Polycomb gene may be forcibly expressed and then the apoptosis suppressor gene may be expressed. As a result, it is possible to obtain the immortalized megakaryocytes in the megakaryocyte inducing step. Further, for example, by halting the forced expression of the immortalized megakaryocytes, the immortalized megakaryocytes can be induced into multinucleated megakaryocytes to produce platelets.

Because the megakaryocyte inducing step, for example, may improve the efficiency to introduce each gene, the megakaryocyte inducing step preferably includes a first expression step of forcibly expressing an oncogene and a Polycomb gene in cells that are less differentiated than the megakaryocytes, a second expression step of forcibly expressing an apoptosis suppressor gene, such as Bcl-xL gene or the like, in the cells that are less differentiated than the megakaryocytes, and a halting step of halting the forced expression.

Forced expression and the halting of forced expression of each gene can be carried out, for example, using a known method such as the methods described in WO 2011/034073, WO 2012/157586, WO 2014/123242 or Reference 7 below, or a method that corresponds to these methods, and are incorporated herein by reference as part of this specification. As a specific example, the forced expression and the halting of the forced expression of each gene can be carried out, for example, using a pharmaceutical agent responsive gene expression induction system. Examples of the gene expression induction system include Tet-on^{®} system, and Tet-off^{®} system. If the Tet-on system is used, for example, in the forced expression step, culturing is carried out in the presence of a pharmaceutical agent for inducing gene expression, such as tetracycline, doxycycline, or the like, and in the forced expression halting step, the culturing is carried out in the absence of the pharmaceutical agent.

Reference 7: Nakamura S et al., "Expandable megakaryocyte cell lines enable clinically applicable generation of platelets from human induced pluripotent stem cells", Cell Stem Cell, 2014, vol.14, No.4, pages 535-548

Then, a culture of the megakaryocytes is produced in the producing step. The producing step can be carried out, for example, by culturing the megakaryocytes in a culture medium. The megakaryocytes may be cultured, for example, on feeder cells, or without feeder cells. The megakaryocytes can be cultured in suspension, and thus the megakaryocytes can be cultured, for example, without the feeder cells. The culture of megakaryocytes contains, for example, platelets.

There is no particular limitation on the culture conditions for the megakaryocytes, and normal culture conditions for the megakaryocytes can be adopted. As specific examples, a culture temperature is, for example, in a range of about 35°C to about 42°C, about 36°C to about 40°C, and about 37°C to about 39°C. The CO₂ concentration is in a range of about 5% to about 15%, for example. The O₂ concentration is, for example, in a range of about 15% to about 25%, and is about 20%. There is no particular limitation on the culturing period, and examples thereof include about 1 day to about 2 weeks, and about 4 to about 8 days.

There is no particular limitation on the culture medium, and examples thereof include known culture media that are suitable for producing platelets from the megakaryocytes and culture media that are similar thereto. As a specific example, the culture medium can be prepared, for example, using a culture medium that is to be used to culture mammalian cells, as a basal culture medium. Examples of the basal medium include single media such as IMDM, Medium 199, Eagle's Minimum Essential Medium (EMEM), αMEM, Dulbecco's modified Eagle's Medium (DMEM), Ham's F12 medium, RPMI1640 medium, Fischer's culture medium, and Neurobasal^{®} Medium (manufactured by Thermo Fisher Scientific), and a mixed medium thereof. The culture medium may contain, for example, serum or blood plasma, or may be a serum-free medium that does not contain serum or blood plasma. The origins of the serum and the blood plasma are preferably the same as the origin of the megakaryocytes. If the megakaryocytes are derived from a human as a specific example, the serum and the blood plasma are each preferably derived from a human.

The culture medium, for example, may contain other components. There is no particular limitation on the other components, and examples thereof include albumin, insulin, transferrin, selenium, fatty acids, trace elements, 2-mercaptoethanol, thiolglycerol, monothioglycerol (MTG), lipids, amino acids (e.g., L-glutamine), ascorbic acid, heparin, non-essential amino acids, vitamins, growth factors, low molecular weight compounds, antibiotics, antioxidants, pyruvic acid, buffers, inorganic salts, and cytokines. The number of types of other components may be, for example, one, or two or more. The cytokine is a substance that promotes differentiation of hematopoietic cells, and specific examples thereof include vascular endothelial cell growth factor (VEGF), thrombopoietin (TPO), various TPO-like active substances, Stem Cell Factor (SCF), ITS (insulin-transferrin-selenite) supplements, ADAM inhibitors, FLT inhibitors, WNT inhibitors, ROCK inhibitors, and aryl hydrocarbon receptor (AhR) inhibitors. The culture medium is preferably IMDM containing, for example, serum, insulin, transferrin, serine, thiolglycerol, ascorbic acid, and TPO. The culture medium may further contain, for example, SCF and further contain heparin. There is no particular limitation on the concentration of the other components. The TPO concentration is, for example, in a range of about 10 ng/ml to about 200 ng/ml, and about 50 ng/ml to about 100 ng/ml. The SCF concentration is, for example, about 10 ng/ml to about 200 ng/ml, and about 50 ng/ml. The heparin concentration is, for example, in a range of about 10 U/ml to about 100 U/ml, and is about 25 U/ml. The culture medium may further contain, for example, a phorbol ester (e.g., phorbol-12-myristate-13-acetate; PMA).

Then, the megakaryocytes or a culture of the megakaryocytes is treated in the treating step. In the treating step, protein is extracted, for example, by destroying the cell membrane of the megakaryocytes or the cells contained in the culture of the megakaryocytes. As a specific example, there is no particular limitation on treatment performed in the treating step, and examples thereof include treatments for changing a density of cell components, such as a concentrating treatment; extraction treatments for extracting cell components, such as a drying treatment, a freezing treatment, a freeze-drying treatment, a solvent treatment, a surfactant treatment, an enzyme treatment, a protein fraction extraction treatment, and a sonication treatment; and disruption treatments such as a homogenization treatment and a pulverization treatment, or the like. The number of types of treatments performed in the treating step may be one, or two or more. Also, in the treating step, the treatment may be performed once, or twice or more.

The concentrating treatment can be carried out, for example, by centrifuging the megakaryocytes or the culture thereof. For example, conditions under which cells or platelets are precipitated can be adopted as the centrifugation condition. The drying treatment can be carried out, for example, by drying the megakaryocytes or the culture thereof using a dry spray, a drum dryer, or the like. The freeze-drying treatment can be carried out, for example, using a freeze-dryer. In the solvent treatment, the solvent is, for example, an organic solvent such as phenol or chloroform; an aqueous solvent such as water, physiological saline, or a buffer solution. If an aqueous solvent is used as the solvent, the solvent treatment is, for example, preferably used in combination with a surfactant treatment, an enzyme treatment, and/or a sonication treatment, which will be described later. The solvent treatment can be carried out, for example, by mixing the megakaryocytes or the culture thereof with the solvent. In the surfactant treatment, examples of the surfactant include ionic surfactants such as sodium lauryl sulfate; non-ionic surfactants such as NP-40, Triton X-100, Tween 20, and n-Dodecyl-β-D-maltopyranoside; zwitterionic surfactants such as CHAPS; and the like. The surfactant concentration is, for example, a concentration at which a cell membrane of the megakaryocytes or cell components in the culture of the megakaryocytes can be destroyed. The surfactant treatment can be carried out, for example, by bringing the megakaryocytes or the culture thereof into contact with the surfactant in an aqueous solvent. The surfactant is brought into contact therewith, for example, at about 0°C to about 10°C. Examples of the aqueous solvent include water, physiological saline, and a buffer solution. Examples of the enzyme used in the enzyme treatment include peptidases and proteases. The enzyme treatment can be carried out, for example, by bringing the megakaryocytes or the culture thereof into contact with the enzyme in the aqueous solvent. The conditions for the enzyme treatment are, for example, conditions under which the enzyme exhibits activity. The protein fraction extraction treatment can be carried out, for example, by subjecting the megakaryocytes or the culture thereof to osmotic shock, freeze-thawing, or the like. The sonication treatment can be carried out, for example, using a sonicator. For example, conditions under which cells are disrupted can be used as the conditions for the sonication treatment.

The treatment conditions and the treatment time in various treatments can be determined, for example, as appropriate according to the type of treatments. Also, in the treating step, the concentration of the total protein in the treated product can be adjusted, for example, by adjusting the amount of the aqueous solvent.

The megakaryocytes or the culture thereof may be processed (pretreated) prior to the treatment. In this case, a culture obtained by culturing the megakaryocytes may be directly used as the culture of the megakaryocytes, or the processed mixture may be used as the culture of the megakaryocytes. The culture may be processed, for example, through removal of a liquid fraction, extraction of a cell component fraction, changing of the composition of the cell components that include platelets. The composition of the cell components can be changed, for example, through removal of cells and/or platelets from the mixture, extraction of cells and/or platelets from the mixture, addition of cells and/or platelets to the mixture, or the like.

If the production method of the present invention includes the pretreatment, the production method of the present invention may include a removing step of removing platelets from the megakaryocytes or the culture thereof. In this case, in the treating step, treatment is carried out using, as the megakaryocytes or the culture thereof, megakaryocytes or a culture thereof from which the platelets are removed, or the removed platelets. The megakaryocytes obtained after the release of the platelets, for example, contain a large amount of bFGF. Thus, with the production method of the present invention, a composition having a high bFGF content can be produced, for example, by removing the platelets. By removing the platelets, the platelets can be separated from other cell fractions. Therefore, the removing step may be referred to, for example, as a platelet separating step or a step of separating platelets from other cell components. In the removing step, a method for separating platelets from the culture of megakaryocytes can be carried out, for example, using a known method such as the method described in WO 2017/065280 (US 2018/282697A), or a method that corresponds to this method, and these methods are incorporated herein by reference as part of this specification.

The platelet removal percentage (separation percentage) in the removing step is, for example, 60% or more, 70% or more, 80% or more, 85% or more, 90% or more, 95% or more, 96% or more, 97% or more, 98% or more, or 99% or more. The platelet removal percentage is, for example, in a range of 60% to 90%.

The production method of the present invention may include a storing step of storing the megakaryocytes or the culture thereof, megakaryocytes or a culture thereof from which the platelets are removed, or the removed platelets. Examples of the storage in the storing step include refrigerated storage (about 1°C to about 10°C) and frozen storage (about -200°C to about -4°C). There is no particular limitation on the storage period in the storing step. The storing step also serves, for example, as a freezing treatment in the treating step, and thus the frozen storage is preferable.

As an example, the production method of the present invention can be carried out as follows. However, the present invention is not limited to the following examples. First, a culture medium containing the megakaryocytes or a culture thereof is subjected to a concentrating treatment through centrifugation so as to concentrate cell components. The centrifugation treatment is carried out, for example, under the conditions under which the cell components precipitate. As specific examples, the centrifugation treatment can be carried out through centrifugation at 1000 to 3000 × g for 5 to 20 minute. Then, after centrifugation is performed, a precipitate is collected, and a freezing treatment is performed by quickly freezing the obtained precipitate. The freezing treatment can be carried out, for example, by bringing the precipitate into contact with liquefied gas such as liquid nitrogen. Further, a surfactant treatment is performed by dissolving the frozen precipitate in an aqueous solvent containing the surfactant. The obtained solution is centrifuged so as to precipitate contaminants. The centrifugation treatment is carried ou, for example, under the conditions with which the contaminants such as cell membrane precipitate. As specific examples, the centrifugation treatment can be carried out through centrifugation at 10000 to 20000 × g for 3 to 10 minutes. Because the protein is present in the supernatant after the centrifugation is performed, the treated product can be obtained by collecting the supernatant as a protein fraction.

### Composition obtained using production method

A composition of the present invention (also referred to as a "second composition") can be obtained by using the production method of the present invention. The second composition of the present invention can be obtained by using the production method according to the present invention, and there is no particular limitation on other constituents and conditions. According to the second composition of the present invention, it is possible to provide, for example, a composition having physiological activity. The second composition of the present invention can, for example, promote cell proliferation. The above descriptions of the composition and the production method of the present invention can be applied to the second composition of the present invention.

### Cell proliferation promoting composition

Another example of the present invention provides a composition capable of promoting cell proliferation. As described above, the cell proliferation promoting composition according to the present invention contains the composition according to the present invention. The proliferation promoting composition of the present invention contains the composition of the present invention, and there is no particular limitation on other constituents and conditions. According to the proliferation promoting composition of the present invention, it is possible, for example, to promote the proliferation of cells, in particular, mesenchymal stem cells. The above descriptions of the composition and the production method of the present invention can be applied to the proliferation promoting composition of the present invention.

The proliferation promoting composition of the present invention may be used *in vitro* or *in vivo.*

If the proliferation promoting composition of the present invention is used *in vitro*, examples of the administration target include cells, tissues, and organs, and examples of the cells include cells collected from an organism and cultured cells.

If the proliferation promoting composition of the present invention is used *in vivo*, examples of the administration target include humans and non-human animals that exclude humans. Examples of the non-human animals include mice, rats, rabbits, dogs, sheep, horses, cats, goats, monkeys, and guinea pigs.

There is no particular limitation on the conditions for using the proliferation promoting composition of the present invention (administration conditions), and an administration form, an administration period, a dosage, and the like can be set, for example, as appropriate according to the type of administration target or the like.

If the proliferation promoting composition of the present invention is used *in vitro*, the proliferation promoting composition of the present invention can be used, for example, by adding this proliferation promoting composition to a culture medium for a target cell. The final concentration of the total protein derived from the proliferation promoting composition of the present invention in the culture medium is in a range of 10 to 1000 µg/ml, 10 to 500 µg/ml, 10 to 320 µg/ml, 10 to 300 µg/ml, or 20 to 300 µg/ml.

If the proliferation promoting composition of the present invention is used *in vivo*, the final concentration thereof can be determined as appropriate dependently on the type of administration target, symptom, age, administration method, and the like. If this composition is administered to a human as a specific example, there is no particular limitation on the daily dose of the proliferation promoting composition and the total amount of protein derived from the proliferation promoting composition, and the daily dosage and the total amount can be set, for example, as appropriate according to the applications thereof. The number of administrations per day is, for example, in a range of 1 to 5 or 1 to 3, or 1, or 2.

There is no particular limitation on the form for administering the proliferation promoting composition of the present invention. If the proliferation promoting composition of the present invention is administered *in vivo*, the proliferation promoting composition may be administered orally or parenterally. Examples of the parenteral administration include intravenous injection (intravenous administration), intramuscular injection (intramuscular administration), transdermal administration, subcutaneous administration, intradermal administration, enteral administration, rectal administration, transvaginal administration, transnasal administration, pulmonary administration, intraperitoneal administration, and local administration.

There is no particular limitation on the dosage form of the proliferation promoting composition of the present invention, and the dosage form thereof can be determined, for example, as appropriate according to the administration form, for example. The dosage form is liquid or solid.

The proliferation promoting composition of the present invention may contain, for example, an additive agent as needed. The additive agent is preferably a pharmaceutically acceptable additive agent or a pharmaceutically acceptable carrier.

### Fibroblast function promoting composition

Another example of the present invention provides a composition capable of promoting a function of fibroblasts. As described above, the fibroblast function promoting composition of the present invention contains the composition of the present invention. The fibroblast function promoting composition of the present invention contains the composition of the present invention, and there is no particular limitation on other constituents and conditions. According to the fibroblast function promoting composition of the present invention, it is possible to promote the function of the fibroblasts. The above descriptions of the composition, the production method, and the proliferation promoting composition of the present invention can be applied to the fibroblast function promoting composition of the present invention.

The fibroblast function promoting composition of the present invention may be used *in vitro* or *in vivo*.

If the fibroblast function promoting composition of the present invention is used *in vitro*, the fibroblast function promoting composition of the present invention can be used, for example, by adding this fibroblast function promoting composition to a culture medium for a target fibroblast. According to the fibroblast function promoting composition of the present invention, the proliferation of the fibroblasts and/or the production of the extracellular matrix by the fibroblasts can be promoted, for example, by maintaining the fibroblasts in a culture medium containing the fibroblast function promoting composition. The final concentration of the total protein of the fibroblast function promoting composition of the present invention in the culture medium is in a range of 10 to 1000 µg/ml, 10 to 500 µg/ml, or 10 to 300 µg/ml.

The above descriptions of the administration target and the administration conditions regarding the proliferation promoting composition of the present invention can be applied to the administration target and the administration conditions regarding the fibroblast function promoting composition of the present invention.

### Composition for promoting healing of skin disorders

Another example of the present invention provides a composition that can be used to promote healing of skin disorders. As described above, the composition for promoting healing of a skin disorder of the present invention (hereinafter also referred to as "healing promoting composition") according to the present invention contains the composition of the present invention. The healing promoting composition of the present invention contains the composition of the present invention, and there is no particular limitation on other constituents and conditions. It is expected that the healing promoting composition of the present invention can promote healing of skin disorders. The above descriptions of the composition, the production method, and the proliferation promoting composition of the present invention can be applied to the healing promoting composition of the present invention.

In the present invention, the skin disorders refer to, for example, a state in which skin is injured or damaged, that is, a state in which the structure of normal skin tissue is impaired or destroyed, and specific examples thereof include skin ulcers, pressure sores, burns, scars, wounds, and skin aging.

The healing promoting composition of the present invention may be used *in vitro* or *in vivo.*

If the healing promoting composition of the present invention is used *in vitro*, the healing promoting composition of the present invention can be used, for example, by adding this healing promoting composition to a culture medium for a target cell. The final concentration of the total protein of the healing promoting composition of the present invention in the culture medium is in a range of 10 to 1000 µg/ml, 10 to 500 µg/ml, or 10 to 300 µg/ml.

The above descriptions of the administration target and the administration conditions regarding the proliferation promoting composition of the present invention can be applied to the administration target and the administration conditions regarding the healing promoting composition of the present invention. The healing promoting composition of the present invention is preferably used in a dosage form such that the healing promoting composition can be administered subcutaneously or transdermally.

### Keratinocyte function promoting composition

Another example of the present invention provides a composition capable of promoting a function of keratinocytes. As described above, the keratinocyte function promoting composition of the present invention contains the composition of the present invention. The keratinocyte function promoting composition of the present invention contains the composition of the present invention, and there is no particular limitation on other constituents and conditions. According to the keratinocyte function promoting composition of the present invention, it is possible to promote the function of the keratinocytes. The above descriptions of the composition, the production method, and the proliferation promoting composition of the present invention can be applied to the keratinocyte function promoting composition of the present invention.

The keratinocyte function promoting composition of the present invention may be used *in vitro* or *in vivo.*

If the keratinocyte function promoting composition of the present invention is used *in vitro*, the keratinocyte function promoting composition of the present invention can be used, for example, by adding this keratinocyte function promoting composition to a culture medium for a target keratinocyte. The keratinocyte function promoting composition of the present invention may be added to a maintenance medium to be used to maintain the keratinocytes, may be added to a differentiation medium for differentiating the keratinocytes into epithelial cells such as epidermal cells, or may be added to both the maintenance medium and the differentiation medium. According to the keratinocyte function promoting composition of the present invention, the proliferation of the keratinocytes, differentiation into the epidermal cells, and/or the induction of the barrier function gene can be promoted, for example, by maintaining the keratinocytes in a culture medium containing the keratinocyte function promoting composition. The final concentration of the total protein of the keratinocyte function promoting composition of the present invention in the culture medium is in a range of 10 to 1000 µg/ml, 10 to 500 µg/ml, or 10 to 300 µg/ml.

The above descriptions of the administration target and the administration conditions regarding the proliferation promoting composition of the present invention can be applied to the administration target and the administration conditions regarding the keratinocyte function promoting composition of the present invention.

As described above, the keratinocyte function promoting composition of the present invention promotes differentiation of the keratinocytes into epithelial cells such as epidermal cells. Thus, it is expected that the keratinocyte function promoting composition of the present invention can be suitably used, for example, as an additive agent in artificial culturing of skin. As described above, the keratinocyte function promoting composition of the present invention also exhibits the fibroblast function promoting activity. Therefore, it is expected that the keratinocyte function promoting composition of the present invention can be suitably used for regeneration of aged skin by promoting the turnover of skin tissue using keratinocyte and fibroblast function promoting activity. Thus, it is expected that the keratinocyte function promoting composition of the present invention can be suitably used, for example, as an external use composition for skin such as cosmetics, an injection for subcutaneous administration, or the like.

### Dermal papilla cell function promoting composition

Another example of the present invention provides a composition capable of promoting a function of dermal papilla cells. As described above, the dermal papilla cell function promoting composition of the present invention contains the composition of the present invention. The dermal papilla cell function promoting composition of the present invention contains the composition of the present invention, and there is no particular limitation on other constituents and conditions. According to the dermal papilla cell function promoting composition of the present invention, it is possible to promote the function of the keratinocytes. The above descriptions of the composition, the production method, and the proliferation promoting composition of the present invention can be applied to the dermal papilla cell function promoting composition of the present invention.

The dermal papilla cell function promoting composition of the present invention may be used *in vitro* or *in vivo.*

If the dermal papilla cell function promoting composition of the present invention is used *in vitro*, the dermal papilla cell function promoting composition of the present invention can be used, for example, by adding this dermal papilla cell function promoting composition to a culture medium for a target dermal papilla cell. According to the dermal papilla cell function promoting composition of the present invention, the proliferation of the dermal papilla cell, and/or the induction of the hair growth promoting gene can be promoted, for example, by maintaining the dermal papilla cells in a culture medium containing the dermal papilla cell function promoting composition. The final concentration of the total protein of the dermal papilla cell function promoting composition of the present invention in the culture medium is in a range of 10 to 1000 µg/ml, 10 to 500 µg/ml, or 10 to 300 µg/ml.

The above descriptions of the administration target and the administration conditions regarding the proliferation promoting composition of the present invention can be applied to the administration target and the administration conditions regarding the dermal papilla cell function promoting composition of the present invention.

### Hair growth promoting composition

Another example of the present invention provides a composition capable of promoting hair growth. As described above, the hair growth promoting composition of the present invention contains the composition of the present invention. The hair growth promoting composition of the present invention contains the composition of the present invention, and there is no particular limitation on other constituents and conditions. According to the hair growth promoting composition of the present invention, it is expected that hair growth can be promoted because the function of the dermal papilla cells is promoted. The above descriptions of the composition, the production method, and the proliferation promoting composition of the present invention can be applied to the dermal papilla cell function promoting composition of the present invention.

In the present invention, "promotion of hair growth" refers to enhancing (increasing, raising) the likelihood of hair growth from the absence of hair, promoting hair growth (e.g., the growth of hair length and/or thickness), and/or lessening (suppressing, reducing) hair loss.

The hair growth promoting composition of the present invention may be used, for example, to prevent, suppress, or stop hair loss. The hair growth promoting composition of the present invention can also be referred to, for example, as a composition for preventing, suppressing, or stopping hair loss.

The hair growth promoting composition of the present invention may be used *in vitro* or *in vivo.*

If the hair growth promoting composition of the present invention is used *in vitro*, the hair growth promoting composition of the present invention can be used, for example, by adding this hair growth promoting composition to a culture medium for a target dermal papilla cell. According to the hair growth promoting composition of the present invention, it is expected that the proliferation of the dermal papilla cells, and/or the induction of the hair growth promoting gene can be promoted, for example, by maintaining the dermal papilla cells in a culture medium containing the composition of the present invention, and thus hair growth is promoted. The final concentration of the total protein of the hair growth promoting composition of the present invention in the culture medium is in a range of 10 to 1000 µg/ml, 10 to 500 µg/ml, or 10 to 300 µg/ml.

The above descriptions of the administration target and the administration conditions regarding the proliferation promoting composition of the present invention and the later-described hair growth promoting method can be applied to the administration target and the administration conditions regarding the hair growth promoting composition of the present invention.

### Method for promoting proliferation of cells

A method for promoting proliferation of cells (also referred to as "proliferation promoting method" hereinafter) according to the present invention involves use of the cell proliferation promoting composition of the present invention. The proliferation promoting method according to the present invention involves the use of the proliferation promoting composition of the present invention, and there is no particular limitation on other steps and conditions. According to the proliferation promoting method of the present invention, it is possible to promote proliferation of cells, in particular, mesenchymal stem cells. The above descriptions of the composition, the production method, the proliferation promoting composition, and the differentiation promoting composition of the present invention can be applied to the proliferation promoting method of the present invention.

The proliferation promoting method of the present invention may be used *in vitro* or *in vivo*, for example.

If the proliferation promoting method of the present invention is carried out *in vitro*, the proliferation promoting method of the present invention includes, for example, a culturing step of culturing cells in the presence of the proliferation promoting composition. For example, the above descriptions of the administration target and the administration conditions regarding the proliferation promoting composition of the present invention can be applied to the administration target and the administration conditions regarding the proliferation promoting composition of the present invention.

### Fibroblast function promoting method

Another example of the present invention provides a method with which the function of fibroblasts can be promoted. A fibroblast function promoting method according to the present invention involves use of the composition of the present invention. The fibroblast function promoting method according to the present invention involves the use of the composition of the present invention, and there is no particular limitation on other steps and conditions. According to the fibroblast function promoting method of the present invention, it is possible to promote the function of the fibroblasts. The above descriptions of the composition, the production method, the proliferation promoting composition, and the fibroblast function promoting composition of the present invention can be applied to the fibroblast function promoting method of the present invention.

The fibroblast function promoting method of the present invention may be *used in vitro* or *in vivo*, for example.

If the fibroblast function promoting method of the present invention is carried out *in vitro*, the fibroblast function promoting method of the present invention includes, for example, a culturing step of culturing fibroblasts in the presence of the composition. The above descriptions of the administration target and the administration conditions regarding the proliferation promoting composition of the present invention can be applied, for example, to the administration target and the administration conditions regarding the composition of the present invention, for example.

### Method for promoting healing of skin disorders

Another example of the present invention provides a method that can be used to promote healing of skin disorders. A method for promoting healing of skin disorders (hereinafter also referred to as "healing promoting method") according to the present invention involves use of the composition of the present invention. The healing promoting method according to the present invention involves the use of the composition of the present invention, and there is no particular limitation on other steps and conditions. According to the healing promoting method of the present invention, it is expected that healing of skin disorders can be promoted. The above descriptions of the composition, the production method, the proliferation promoting composition, and the composition for promoting healing of skin disorders of the present invention can be applied to the healing promoting method of the present invention.

The method for promoting healing of skin disorders of the present invention can also be referred to, for example, as a method for curing skin disorders or a method for treating skin disorders.

The healing promoting method according to the present invention may include, for example, an administering step of administering the composition of the present invention to a subject. Examples of the subject include a patient having a skin disorder and a patient who may have a skin disorder.

The healing promoting method of the present invention may be carried out *in vitro* or *in vivo*, for example.

If the healing promoting method of the present invention is carried out *in vitro*, the healing promoting method of the present invention includes, for example, a culturing step of culturing cells relating to skin, such as keratinocytes, fibroblasts, and the like, in the presence of the composition. The above descriptions of the administration target and the administration conditions regarding the proliferation promoting composition of the present invention can be applied, for example, to the administration target and the administration conditions regarding the composition of the present invention.

### Keratinocyte function promoting method

Another example of the present invention provides a method with which the function of keratinocytes can be promoted. A keratinocyte function promoting method according to the present invention involves use of the composition of the present invention. The keratinocyte function promoting method according to the present invention involves the use of the composition of the present invention, and there is no particular limitation on other steps and conditions. According to the keratinocyte function promoting method of the present invention, it is possible to promote the function of the keratinocytes. The above descriptions of the composition, the production method, the proliferation promoting composition, and the keratinocyte function promoting composition of the present invention can be applied to the keratinocyte function promoting method of the present invention.

The keratinocyte function promoting method of the present invention may be used, for example, *in vitro* or *in vivo.*

If the keratinocyte function promoting method of the present invention is carried out *in vitro*, the keratinocyte function promoting method of the present invention includes, for example, a culturing step of culturing keratinocytes in the presence of the composition. The above descriptions of the administration target and the administration conditions regarding the proliferation promoting composition of the present invention can be applied, for example, to the administration target and the administration conditions regarding the composition of the present invention.

### Dermal papilla cell function promoting method

Another example of the present invention provides a method with which the function of dermal papilla cells can be promoted. A dermal papilla cell function promoting method according to the present invention involves use of the composition of the present invention. The dermal papilla cell function promoting method according to the present invention involves the use of the composition of the present invention, and there is no particular limitation on other steps and conditions. According to the dermal papilla cell function promoting method of the present invention, it is possible to promote the function of the dermal papilla cells. The above descriptions of the composition, the production method, the proliferation promoting composition, and the dermal papilla cell function promoting composition of the present invention can be applied to the dermal papilla cell function promoting method of the present invention.

The dermal papilla cell function promoting method of the present invention may be carried out, for example, *in vitro* or *in vivo.*

If the dermal papilla cell function promoting method of the present invention is carried out *in vitro*, the dermal papilla cell function promoting method of the present invention includes, for example, a culturing step of culturing dermal papilla cells in the presence of the composition. The above descriptions of the administration target and the administration conditions regarding the proliferation promoting composition of the present invention can be applied, for example, to the administration target and the administration conditions regarding the composition of the present invention.

### Hair growth promoting method

Another example of the present invention provides a method with which hair growth can be promoted. A hair growth promoting method according to the present invention involves use of the composition of the present invention. The hair growth promoting method according to the present invention involves the use of the composition of the present invention, and there is no particular limitation on other steps and conditions. According to the hair growth promoting method of the present invention, it is expected that hair growth can be promoted. The above descriptions of the composition, the production method, the proliferation promoting composition, and the hair growth promoting composition of the present invention can be applied to the hair growth promoting method of the present invention.

The hair growth promoting method of the present invention can also be referred to, for example, as a method for curing alopecia or a method for treating thinning hair. Also, the hair growth promoting method of the present invention may be used, for example, to prevent, suppress, or stop hair loss. In this case, the hair growth promoting method of the present invention can also be referred to as a method, for example, for preventing, suppressing, or stopping hair loss.

The hair growth promoting method according to the present invention may, for example, include an administering step of administering the composition of the present invention to a subject. Examples of the subject include, for example, patients with alopecia and patients who may have alopecia. Examples of the alopecia patient include male pattern alopecia, female pattern alopecia, and alopecia after anticancer therapy. Examples of the patient that may lose hair include patients before an anticancer therapy and patients who have genetic alopecia before hair loss starts.

The hair growth promoting method of the present invention may be carried out, for example, *in vitro* or *in vivo.*

If the hair growth promoting method of the present invention is carried out *in vitro*, the hair growth promoting method of the present invention includes, for example, a culturing step of culturing cells relating to hair growth, such as dermal papilla cell, in the presence of the composition. The above descriptions of the administration target and the administration conditions regarding the proliferation promoting composition of the present invention can be applied, for example, to the administration target and the administration conditions regarding the composition of the present invention.

### Use of composition

The present invention relates to a composition for use for promoting cell proliferation that contains, as an active ingredient, a treated product of megakaryocytes or a culture of the megakaryocytes, or to use thereof. Further, the present invention relates to a composition for use for promoting the function of fibroblasts that contains, as an active ingredient, a treated product of megakaryocytes or a culture of the megakaryocytes, or to use thereof. The present invention relates to a composition for use for promoting healing of skin disorders that contains, as an active ingredient, a treated product of megakaryocytes or a culture of the megakaryocytes, or to use thereof. The present invention relates to a composition for use for promoting the function of keratinocytes that contains, as an active ingredient, a treated product of megakaryocytes or a culture of the megakaryocytes, or to use thereof. The present invention relates to a composition for use for promoting the function of dermal papilla cells that contains, as an active ingredient, a treated product of megakaryocytes or a culture of the megakaryocytes, or to use thereof. The present invention relates to a composition for use for promoting hair growth that contains, as an active ingredient, a product treated product of megakaryocytes or a culture of the megakaryocytes, or to use thereof. The present invention relates to use of a composition for producing a cell proliferation promoting composition that contains, as an active ingredient, a treated product of megakaryocytes or a culture of the megakaryocytes. Also, the present invention relates to use of a composition for producing a fibroblast function promoting composition that contains, as an active ingredient, a treated product of megakaryocytes or a culture of the megakaryocytes. The present invention relates to use of a composition for producing a composition for promoting healing of skin disorders that contains, as an active ingredient, a treated product of megakaryocytes or a culture of the megakaryocytes. The present invention relates to use of a composition for producing a keratinocyte function promoting composition that contains, as an active ingredient, a treated product of megakaryocytes or a culture of the megakaryocytes. The present invention relates to use of a composition for producing a dermal papilla cell function promoting composition that contains, as an active ingredient, a treated product of megakaryocytes or a culture of the megakaryocytes. The present invention relates to use of a composition for producing a hair growth promoting composition that contains, as an active ingredient, a treated product of megakaryocytes or a culture of the megakaryocytes.

### Examples

The following describes the present invention in detail using Examples, but the present invention is not limited to aspects described in Examples.

### Example 1

The composition according to the present invention was produced, and it was confirmed that the composition contains a growth factor and a growth factor receptor, and that the composition had cell proliferation promoting activity.

### (1) Production of immortalized megakaryocytes

Immortalized megakaryocytes were produced using the following procedure.

### (1-1) Preparation of hematopoietic progenitor cells from iPS cells.

Culturing for differentiation from human iPS cells (TKDN SeV2 and NIH5: human fetal dermal fibroblast-derived iPS cells established using Sendai virus) into blood cells was carried out according to the method described in Reference 8 below. Specifically, Hematopoietic Progenitor Cells (HPCs) were produced by co-culturing human ES/iPS cell colonies with C3H10T1/2 feeder cells in the presence of 20 ng/ml VEGF (manufactured by R&D SYSTEMS) for 14 days. The culture conditions were set to be 37°C, 20% O₂, and 5% CO₂ (the same conditions were used unless otherwise specified).

Reference 8: Takayama N. et al., "Transient activation of c-MYC expression is critical for efficient platelet generation from human induced pluripotent stem cells", J. Exp. Med., 2010, vo.13, pages 2817-2830

### (1-2) Gene transfer system

A lentiviral vector system was used as a gene transfer system. A lentiviral vector is a Tetracycline controlled Tet-on^{®} gene expression induction system vector. This system was produced through recombination of the mOKS cassette of LV-TRE-mOKS-Ubc-tTA-I2G (Reference 9 below) with c-MYC, BMI1, or BCL-xL. The vector into which c-MYC was introduced was LV-TRE-c-Myc-Ubc-tTA-I2G, the vector into which BMI1 was introduced was LVTRE-BMI1-Ubc-tTA-I2G, and the vector into which BCL-xL was introduced was LV-TRE-BCL-xL-Ubc-tTA-I2G. c-MYC, BMI1, and BCL-xL viruses were produced through gene transfer into 293T cells using the lentiviral vector. By infecting the cells of interest with the resulting virus, c-MYC, BMI1, and BCL-xL genes are respectively introduced into the genomic sequences of the cells of interest. These genes, which have been stably introduced into the genomic sequences, can be forcibly expressed by adding doxycycline (clontech#631311) to a culture medium.

Reference 9: Kobayashi, T. et al., "Generation of rat pancreas in mouse by interspecific blastocyst injection of pluripotent stem cells", Cell, 2010, vol. 142, No. 5, pages 787-799

### (1-3) Infection of hematopoietic progenitor cells with c-MYC and BMI1 viruses

HPCs obtained by using the above method in (1-1) were seeded at 5×10⁴ cells/well on a 6-well plate where C3H10T1/2 feeder cells were pre-seeded, and c-MYC and BMI1 were forcibly expressed using the lentivirus method in which the BMI1 virus and the c-MYC virus were used. At this time, 6 wells were used for each cell line. Specifically, virus particles were added to a culture medium such that the MOI (multiplicity of infection) was 20, and cells were infected through spin infection (centrifugation at 32°C and 900 rpm for 60 minutes). The spin infection was carried out twice every 12 hours. The used culture medium was prepared as follows: a culture medium (differentiation medium hereinafter) was prepared by adding Human thrombopoietin (TPO) (R&D SYSTEMS), Human Stem Cell Factor (SCF) (R&D SYSTEMS), and Doxycycline (Dox, clontech #631311) to a basal medium (IMDM (Iscove's Modified Dulbecco's Medium) (Sigma-Aldrich) that contained 15% Fetal Bovine Serum (GIBCO), 1% Penicillin-Streptomycin-Glutamine (GIBCO), 1% Insulin, Transferrin, Selenium Solution (ITS-G) (GIBCO), 0.45 mmol/l 1-Thioglycerol (Sigma-Aldrich), and 50 µg/ml L-Ascorbic Acid (Sigma-Aldrich)), such that the concentration of TPO was 50 ng/ml, that the concentration of SCF was 50 ng/ml, and that the concentration of Dox was 2 µg/ml, and then Protamine was added to the differentiation medium such that the final concentration of Protamine was 10 µg/ml.

### (1-4) Production and maintenance culture of megakaryocyte self-proliferation strain

Megakaryocyte self-proliferation strains were produced by culturing HPCs into which the c-MYC gene and the BMI1 gene were introduced respectively, as follows, where the day when the c-MYC virus infection and the BMI1 virus infection were carried out using the method in (1-3) above was set to the 0th day of the infection. The c-MYC gene and the BMI1 gene were forcibly expressed by adding DOX to the culture medium such that the concentration of DOX was 1 µg/ml.

### • 2nd day of infection to 11th day of infection

On the 2nd day of the infection, the blood cells infected with the viruses obtained by using the above method were collected through pipetting, centrifuged at 1200 rpm for 5 minutes to remove the supernatant, suspended in a new differentiation medium, and seeded on new C3H10T1/2 feeder cells (6-well plate). Subculturing was carried out by performing the same operation on the 9th day of the infection. At the time of re-seeding, the number of cells was measured, and the cells were seeded on the C3H10T1/2 feeder cells such that the number of cells was 1×10⁵ cells/2 ml/well (6-well plate).

### • 12th day of infection to 13th day of infection

The operation that was same as on the 2nd day of the infection was carried out. After the number of cells was measured, the cells were seeded on the C3H10T1/2 feeder cells (100 mm dish) such that the number of cells reached 3×10⁵ cells/10 ml/100 mm dish.

### • 14th day of infection

The blood cells infected with the viruses were collected, and the blood cells were reacted with 2 µl of anti-human CD41a-APC antibody (BioLegend), 1 µl of anti-human CD42b-PE antibody (eBioscience), and 1 µl of anti-human CD235ab-pacific blue (BioLegend) antibody, in 1.0×10⁵ cells. After the completion of the reaction, FACS Verse ^{™} (BD Biosciences) was used for an analysis. On the 14th day of the infection, cells having a CD41a positive rate of 50% or more were used as megakaryocyte self-proliferation strains.

### (1-5) Infection of megakaryocyte self-proliferation strain with BCL-xL virus

The BCL-xL gene was introduced into the megakaryocyte self-proliferation strain on the 14th day of the infection using the lentivirus method in which BCL-xL virus was used. The virus particles were added to a culture medium such that the MOI was 10, and the cells were infected through spin infection (centrifugation at 32°C and 900 rpm for 60 minutes). The BCL-xL gene was forcibly expressed by adding DOX to the culture medium such that the concentration of DOX was 1 µg/ml.

### (1-6) Production and maintenance culture of immortalized megakaryocyte strain

### • 14th day of infection to 18th day of infection

The megakaryocyte self-proliferation strain into which the BCL-xL gene obtained by using the method in (1-5) above was introduced was collected and centrifuged at 1200 rpm for 5 minutes. After the centrifugation was performed, precipitated cells were suspended in a new differentiation medium and seeded on new C3H10T1/2 feeder cells such that the number of cells was 2×10⁵ cells/2 ml/well (6-well plate).

### • 18th day of infection: Subculture

The megakaryocyte self-proliferation strain into which the BCL-xL gene was introduced was collected, the number of cells was measured, and the cells were seeded to 3×10⁵ cells/10 ml/100 mm dish.

### • 24th day of infection: Subculture

The megakaryocyte self-proliferation strain into which the BCL-xL gene was introduced was collected, the number of cells was measured, and the cells were seeded to 1×10⁵ cells/10 ml/100 mm dish. Hereinafter, subculturing was performed in the same manner every 4 to 7 days, and maintenance culturing was performed. Note that at the time of subculturing, the cells were suspended in a new differentiation medium and seeded.

On the 24th day of the infection, the megakaryocyte self-proliferation strain into which the BCL-xL gene was introduced were collected, and the cells were immunostained using 2 µl of anti-human CD41a-APC antibody (BioLegend), 1 µl of anti-human CD42b-PE antibody (eBioscience), and 1 µl of anti-human CD235ab-Pacific Blue (Anti-CD235ab-PB; BioLegend) antibody, in 1.0×10⁵ cells, and an analysis was performed using FACS Verse ^{™}. Then, on the 24th day of the infection, cell lines having a CD41a positive rate of 50% or more were used as immortalized megakaryocyte cell lines. These cells, which were able to proliferate for 24 days or more after the infection, were used as immortalized megakaryocyte cell lines SeV2-MKCL and NIH5-MKCL.

The obtained SeV2-MKCL and NIH5-MKCL were statically cultured in a 10-cm dish (10 ml/dish). With regard to a culture medium, IMDM was used as a basal medium, and the following components were added (the concentration refers to the final concentration). The culture conditions were set to 37°C and 5% CO₂.
FBS (Sigma #172012 lot.12E261) 15%
L-Glutamine (Gibco #25030-081) 2 mmol/l
ITS (Gibco #41400-045) 100X
MTG (monothioglycerol, sigma #M6145-25ML) 450 µmol/l
Ascorbic acid (sigma #A4544) 50 µg/ml
Puromycin (sigma #P8833-100MG) 2 µg/ml
SCF (Wako Pure Chemical Corp. #193-15513) 50 ng/ml
TPO-like active substance 200 ng/ml

### (2) Production of culture of megakaryocytes

Forced expression was halted through culturing in a culture medium without DOX. Specifically, the immortalized megakaryocyte cell lines (SeV2-MKCL and NIH5-MKCL) obtained by using the method in (1) above were washed with PBS(-) twice, and suspended in a platelet production medium. The cell seeding density was 1.0×10⁵ cells/ml.

With regard to the platelet production medium, IMDM was used as a basal medium, and the following components were added (the concentration refers to the final concentration).
human plasmA 6%
L-Glutamine (Gibco #25030-081) 4 mmol/l
ITS (Gibco #41400-045) 100X
MTG (monothioglycerol, sigma #M6145-25ML) 450 µmol/l
Ascorbic acid (sigma #A4544) 50 µg/ml
SCF (Wako Pure Chemical Corp. #193-15513) 50 ng/ml
TPO-like active substance 200 ng/ml
ADAM inhibitor 15 µmol/l
GNF351 (Calbiochem #182707) 500 nmol/LY39983 (Chemscene LLC #CS-0096) 500 nmol/l
Urokinase 5 U/ml
Low molecular weight heparin (SANOFI, Clexane) 1 U/ml

Then, a culture of the megakaryocytes was produced through culturing for 6 days in the platelets production medium so as to produce platelets.

### (3) Production of purified platelets

With regard to the culture of the megakaryocytes obtained in (2) above, platelets were produced (purified) using the following procedure. Note that the same purification was performed twice.

### (3-1) Concentrating of culture of megakaryocytes

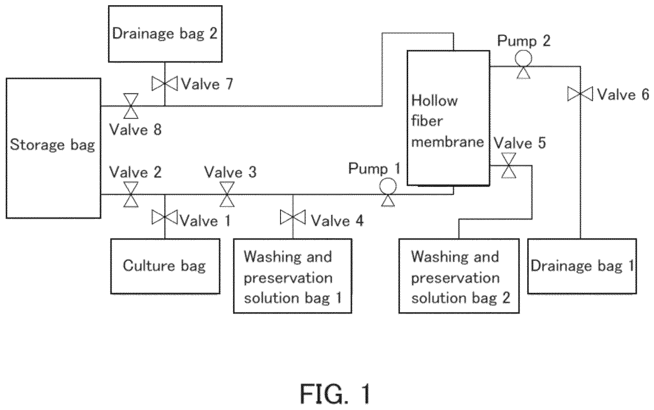

The culture of the megakaryocytes obtained in (2) above was introduced into a culture bag. As shown in FIG. 1, the culture bag was then connected to a concentrating system. In FIG. 1, washing and preservation solution bags 1 and 2 contain a washing and preservation solution. The washing and preservation solution prepared by adding 20% ACD and 2.5% human serum albumin to a BICANATE Injection (BICARBON Injection manufactured by Otsuka Pharmaceutical Co., Ltd.), and adjusting the pH thereof to pH 7.2 using NaOH, was used. The culture of megakaryocytes was concentrated using a hollow fiber membrane (Plasmaflo OP manufactured by Asahi Kasei Medical Co., Ltd.) according to Table 1 below, and the obtained concentrate of the culture of megakaryocytes was collected in a storage bag.

**Table 1**

| Procedures | Release Valve | Pump 1 (rpm) | Pump 2 (rpm) | Contents |
|---|---|---|---|---|
| 1 | 2,3,4 | - | - | Introducing 50 ml of the washing and preservation solution from washing and preservation solution bag 1 |
| 2 | 1,3,4 | - | - | Introducing 50 ml of the washing and preservation solution from washing and preservation solution bag 1 |
| 3 | 5, 6 | - | 100 | Introducing 500 ml of the washing and preservation solution from washing and reservation solution bag 2, and operating pump 2 for 1 minute |
| 4 | 4, 7 | 50 | - | Introducing 500 ml of the washing and preservation solution from washing and preservation solution bag 1, and operating pump 1 for 1 minute |
| 5 | 1,3,6, 8 | 100 | 100 | Introducing the culture into the hollow fiber membrane |
| 6 | 2, 3, 6, 8 | 100 | 100 | Introducing the concentrate in a storage bag |
| 7 | 2,3,4 | - | - | Introducing 50 ml of the washing and preservation solution from washing and preservation solution bag 1 |
| 8 | 4, 8 | 50 | - | Introducing 500 ml of the washing and preservation solution from washing and preservation solution bag 1 |

### (3-2) Centrifugation of platelets

First, a waste liquid bag of an ACP215 disposable set was replaced with a collection bag using a sterile joining device. A High-calic IVH bag (Terumo HC-B3006A) was used as the collection bag. Then, a 10% amount of an ACD-A solution (manufactured by Terumo Corp.) was added to the concentrate of the megakaryocyte culture. After the above addition, the concentrate to which the ACD-A solution was added was injected into a cell bag. The High-calic IVH bag (Terumo HC-B3006A) was used as the cell bag.

Then, a sterile joining device was used to join the cell bag containing the culture to which the ACD-A solution was added, to the ACP215 disposable set. Then, the ACP215 was started in the service mode and the rotational speed was set to 2500 rpm (350 × g). The ACP215 was started, and the culture in the cell bag was introduced into a separation bowl at about 100 ml/min. The liquid component flowing out of the separation bowl was collected in a collection bag. After the entire amount of the culture in the cell bag was introduced into the separation bowl, 500 ml of the washing and preservation solution was introduced into the separation bowl. After the washing and preservation solution was introduced into the separation bowl, the centrifugation was stopped, and the collection bag containing collected liquid (the collected liquid components that included platelets) was separated therefrom using a tube sealer.

The sterile joining device was used to join the collection bag containing the collected liquid (containing platelets) to a new ACP215 disposable set. The ACP215 was started in the normal mode. WPC was selected for the program setting, and the ACP215 disposable set to which the collection bag was joined was set according to device instructions. Note that the collection bag containing the collected liquid was installed on the stand.

Then, the centrifugation speed of the ACP215 was changed to 5000 rpm (1398.8 × g), and centrifugation was started. When the collected liquid started to be introduced into the separation bowl, the automatic injection was changed to a manual injection. Specifically, the collected liquid was introduced into the separation bowl at an introduction speed of about 100 ml/min. After the entire collected liquid was added to the separation bowl, 500 ml of the washing and preservation solution was added thereto.

### (3-3) Washing of platelets

Washing was performed using 2000 ml of the washing and preservation solution, according to the program of the ACP215.

### (3-4) Collection of platelets

200 ml of the washed platelets was collected into a platelet product bag according to the program of the ACP215.

### (3-5) Separation of platelets

With regard to the platelet product bag, the platelets were separated using a usual method of using the hollow fiber membrane, and were collected into the collection bag.

### (4) Production of extract

The immortalized megakaryocyte cell line obtained by using the method in (1) above, the platelets collected in the platelet product bag obtained in (3-5) above, and the culture of the megakaryocytes that was collected in a drainage bag and from which the platelets were removed (hereinafter also collectively referred to as a "raw material") were used as the megakaryocytes or the culture thereof. Four separately prepared samples (platelet-removed megakaryocyte cultures 1 to 4) were used as the culture of the megakaryocytes from which platelets were removed.

Each raw material was washed twice with a washing solution. The washing solution was prepared by adding the ACD-A solution to the BICANATE Injection to about 20 (v/v)%, and adjusting the pH of the solution to a pH range of 7.0 to 7.4 using NaOH and used. After each raw material was washed, the washed raw material was centrifuged under conditions of 2000 × g at room temperature (about 25°C) for 10 minutes. After the centrifugation, the precipitate was collected, and the precipitate was frozen using liquid nitrogen. Then, a cell lysis buffer was added to the frozen precipitate, and the precipitate was lysed through shaking at 50 rpm and 4°C for 30 minutes. Protease Inhibitor Cocktail (Cat.No.: AA-PI manufactured by RayBiotech) was added to a commercially available cell lysis buffer (2× Cell Lysis Buffer, Cat.No.: AA-LYS manufactured by RayBiotech), and the resulting buffer was used as the cell lysis buffer.

The obtained cell lysate was centrifuged under conditions of 14000 × g at 4°C for 5 minutes. After the centrifugation, the supernatant was collected as a treated product in Examples. The total protein concentration of the treated product obtained from each raw material was measured using Pierce ^{™} BCA Protein Assay Kit (manufactured by Thermo Fisher Scientific). As a result, when the immortalized megakaryocyte cell line was used, 2.604 mg of the total protein was extracted from 2.2×10⁷ cells. Also, when the platelets were used, 1.187 mg of the total protein was extracted from 2.5×10⁸ cells. Furthermore, when the platelet-removed megakaryocyte cultures 1 to 4 were used, 2.8 mg, 5.944 mg, 3.6 mg, and 4.496 mg of the total protein were extracted respectively from 1.97×10⁸ cells, 5.25×10⁸ cells, 3.64×10⁸ cells, and 6.22×10⁸ cells. Then, the total protein concentration was adjusted to 5 mg/ml, and then, with regard to each treated product, the concentrations of the growth factor (bFGF, IGFBP-1, IGFBP-2, PIGF, VEGF, GDF-15, AR, and BMP-7, HGF) and the growth factor receptor (SCFR, EGFR, and VEGFR2) were measured using Quantibody^{®} Human Growth Factor Array 1 (manufactured by RayBiotech). These results are shown in Table 2 below. Note that the results shown in Table 2 can be said to be the concentration in 5 mg of the total protein.

**Table 2**

| | Immortalized megakaryocytes | Platelets | Platelet-removed megakaryocyte culture 1 | Platelet-removed megakaryocyte culture 2 | Platelet-removed megakaryocyte culture 3 | Platelet-removed megakaryocyte culture 4 |
|---|---|---|---|---|---|---|
| AR | 1.22 | 3.68 | 5.92 | 54.30 | 15.63 | 49.93 |
| bFGF | 12095.43 | 8204.79 | 71688.60 | 64295.53 | 67623.46 | 58957.22 |
| BMP-7 | 7990.20 | 5963.72 | 4411.27 | 325.58 | 71.43 | 157.92 |
| GDF-15 | 7301.68 | 9028.55 | 13392.39 | 22623.63 | 19756.97 | 17166.80 |
| IGFB P-1 | 12.09 | 4.87 | 31.32 | 38.13 | 194.83 | 18.03 |
| IGFB P-2 | 49374.40 | 435632.4 9 | 341979.83 | 168537.40 | 194034.44 | 26793.87 |
| PIGF | 26.58 | 6.60 | 71.01 | 44.90 | 23.52 | 66.87 |
| SCF | 2898.13 | 35.37 | 61.19 | 71.34 | 187.32 | 281.21 |
| VEGF | 228.55 | 472.63 | 1434.18 | 1218.30 | 1321.43 | 1299.05 |
| SCF R | 9440.46 | 953.60 | 2277.34 | 3784.83 | 4437.62 | 3593.33 |
| EGF R | 10.44 | 164.37 | 202.28 | 169.70 | 109.56 | 5.04 |
| VEGF R2 | 134.11 | 343.16 | 522.28 | 1185.16 | 1057.00 | 987.55 |

| | | | | | | |
|---|---|---|---|---|---|---|
| Unit: pg/mL | | | | | | |

### (5) Confirmation of cell proliferation promoting activity

Human adipose tissue-derived mesenchymal stem cells were seeded in a 10-cm dish to be 2.4×10⁵ to 4.8×10⁵ cells/10 ml culture medium/dish. Note that commercially available human adipose tissue-derived mesenchymal stem cells (Cat. No.: C-12977 manufactured by Takara Bio) were subcultured twice, and the collected cells were used as the mesenchymal stem cells. The culture medium was prepared by thawing the frozen composition prepared from the platelet-removed megakaryocyte culture 2 and adding the composition to the Mesenchymal Stem Cell Growth Medium 2 (Cat. No.: C-28009 manufactured by Takara Bio). The composition was added such that the composition-derived total protein concentration in the culture medium was a predetermined concentration (0, 125, 250, or 500 µg/ml). Note that the culture medium was a maintenance medium.

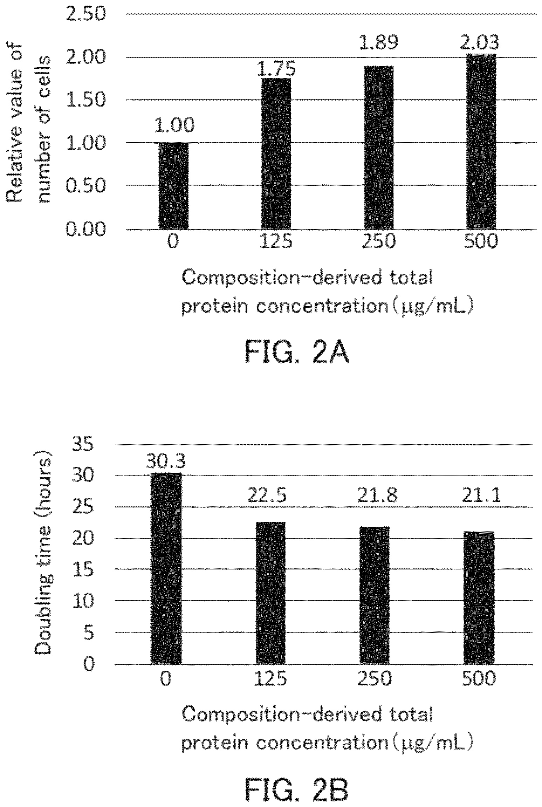

After the seeding, the mesenchymal stem cells were cultured under a wet condition of 37°C and 5% CO₂ for 3 days. After the culturing, the mesenchymal stem cells were collected, and seeded under the same conditions, and cultured again under the wet condition of 37°C and 5% CO₂ for 3 days. Then, the cultured mesenchymal cells were collected, and the number of cells was counted. A relative value of the number of the collected cells was calculated using the number of cells at the time of the seeding as the reference (1), and this value was used as a relative value of the proliferative activity. Also, the time required for a cell to proliferate once (doubling time) was calculated based on the number of the cells at the time of the seeding and the number of the collected cells. These results are shown in FIG. 2.

FIG. 2 is a graph showing the cell proliferative activity. FIG. 2(A) shows proliferative activity, and FIG. 2(B) shows the doubling time. In FIG. 2(A), the composition-derived total protein concentration is shown on the horizontal axis, and the relative value of the proliferative activity is shown on the vertical axis. In FIG. 2(B), the composition-derived total protein concentration is shown on the horizontal axis, the doubling time is shown on the vertical axis, and numerical values in FIG. 2(B) indicate the doubling time. As shown in FIG. 2(A), when the composition of the present invention was added, the proliferative activity of the mesenchymal stem cells increased depending onthe composition-derived total protein concentration. Further, although not shown, with regard to the mesenchymal stem cells to which the composition was not added, the doubling time after the third subculturing was 21 hours. As shown in FIG. 2(B), when the composition of the present invention was not added, the doubling time of the mesenchymal stem cells extended to about 1.5 times the doubling time needed when the composition was added. In contrast, as shown in FIG. 2(B), when the composition of the present invention was added, the time required for mesenchymal stem cells to proliferate once was shortened depending on the composition-derived total protein concentration. Further, when the cells were cultured in a culture medium to which the composition of the present invention was not added, the doubling time of mesenchymal stem cells extended as the number of subculturing increased. In contrast, when the composition of the present invention was added, extension of the doubling time was substantially suppressed. Therefore, it was found that the composition of the present invention exhibits the cell proliferation promoting activity and can suppress a deterioration in the proliferative activity.

### Example 2

It was confirmed that the composition of the present invention had cell proliferation promoting activity.

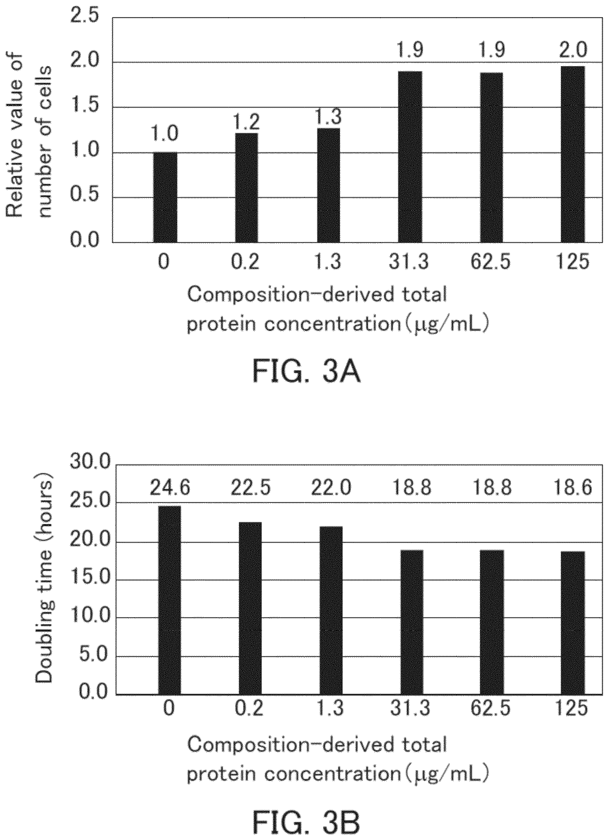

The mesenchymal stem cells were subcultured twice, collected, and cryopreserved. The proliferative activity and the doubling time were calculated in the same manner as in Example 1(5), except that the cryopreserved cells that were thawed and subcultured once were added such that the composition-derived total protein concentration in the maintenance medium became a predetermined concentration (0, 0.2, 1.3, 31.3, 62.5, or 125 µg/ml). These results are shown in FIG. 3.

FIG. 3 is a graph showing the cell proliferative activity. FIG. 3(A) shows proliferative activity, and FIG. 3(B) shows the doubling time. In FIG. 3(A), the composition-derived total protein concentration is shown on the horizontal axis, and the relative value of the proliferative activity is shown on the vertical axis. In FIG. 3(B), the composition-derived total protein concentration is shown on the horizontal axis, and the doubling time is shown on the vertical axis. As shown in FIG. 3(A), when the composition of the present invention was added, the proliferative activity of the mesenchymal stem cells increased depending on the composition-derived total protein concentration, and in particular, when the total protein concentration was 31.3 µg/ml or more, the proliferative activity of mesenchymal stem cells significantly increased. Further, although not shown, with regard to the mesenchymal stem cells to which the composition was not added, the doubling time after the third subculturing was 17 hours. As shown in FIG. 3(B), when the composition of the present invention was not added, the doubling time of the mesenchymal stem cells extended to about 1.5 times the doubling time needed when the composition was added. In contrast, as shown in FIG. 3(B), when the composition of the present invention was added, the time required for mesenchymal stem cells to proliferate once was shortened depending on the composition-derived total protein concentration. In particular, when the total protein concentration was 31.3 µg/ml or more, the time required for the mesenchymal stem cells to proliferate once was significantly shortened. Further, when the cells were cultured in a culture medium to which the composition of the present invention was not added, the doubling time of mesenchymal stem cells extended as the number of subculturing increased. In contrast, when the composition of the present invention was added, extension of the doubling time was substantially suppressed, and this effect was remarkable when the composition-derived total protein concentration was 31.3 µg/ml or more. Therefore, it was found that the composition of the present invention exhibits the cell proliferation promoting activity and can suppress a deterioration in the proliferative activity.

### Example 3

It was confirmed that the composition of the present invention had fibroblast function promoting activity.

### (1) Culturing of fibroblasts

Normal human dermal fibroblasts (nHDF, Cat. No.: KF-4109 manufactured by KURABO) were initiated using a growth medium in a T-75 flask (manufactured by Sumilon), and were cultured using a CO₂ incubator under predetermined culture conditions (under wet conditions at 5% CO₂ and 37°C, and the same applies to the following). The DMEM medium (Cat. No.: 08456-65 manufactured by Nacalai tesque) containing 10% fetal bovine serum (FBS, Cat. No.: 172012 manufactured by Sigma-Aldrich) and 1% penicillin streptomycin (Cat. No.: 15140-122 manufactured by Thermo Fisher Scientific) was used as the growth medium. During the culturing, the culture medium was replaced every 1 to 2 days. When the cells reached about 80% confluent, the cells were collected and used in the following tests. The cells were subcultured as follows. First, after the cells was washed with phosphate buffer (PBS) (-/-) (Cat. No.: 14249-95 manufactured by Nacalai tesque), the cells were detached using a dissociation solution (2.5 g/l-Trypsin/1 mmol/l-EDTA Solution, with Phenol Red (0.25% Trypsin-EDTA), Cat. No.: 32777-44 manufactured by Thermo Fisher Scientific), and trypsin was neutralized by adding the growth medium. Then, the cell suspension was collected in a 15 ml-centrifuge tube and centrifuged (room temperature, 1000 rpm, 5 minutes) using a centrifuge (multipurpose cooled centrifuge, Cat. No.: CAX-571 manufactured by TOMY). After the centrifugation, the supernatant was removed, a new growth medium was added, cells were stirred, and a number of living cells was counted using the trypan blue assay. The cell concentration was adjusted to a desired cell concentration using the growth medium, and the cells were seeded in an incubator used in the subsequent tests.

### (2) Confirmation of fibroblast proliferation promoting activity

The cells were seeded on a 96-well plate (Cat. No.: MS-8096F manufactured by Sumilon) at a density of 5×10³ cells/0.1 ml/well and cultured for 1 day under the above-described culture conditions. After the culturing, the culture medium was replaced with a DMEM medium containing a composition (the total protein concentration was 5 mg/ml, the same being applied to Examples 4 and 5) prepared from the platelet-removed megakaryocyte culture (MDF) or the platelets (PLT) so as to achieve a predetermined concentration (each test substance treatment concentration: 0.5%, 1%, 2.5%, 5%, or 10%). Thus, the concentrations of the total protein derived from the composition in the culture solutions were 25 µg/ml (0.5%), 50 µg/ml (1%), 125 µg/ml (2.5%), 250 µg/ml (5%), and 500 µg/ml (10%) (the same being applied to the followings). Note that two types (iMDF1, iMDF2) of compositions prepared from the platelet-removed megakaryocyte culture were used. After the culture medium was replaced, culturing was performed for 48 hours.

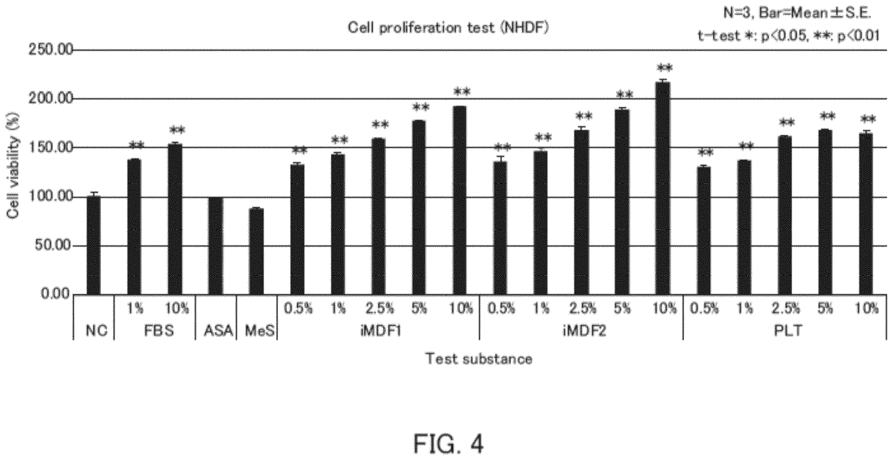

After the culturing, the proliferative activity was measured by measuring a number of living cells in each well using the WST-8. First, the culture medium was replaced with the DMEM culture medium containing a 10% color reagent (Cell Count Reagent SF, Cat. No.: 07553-15 manufactured by Nacalai tesque), and the cells were incubated under the culture conditions. The amount of change in absorbance (450 nm) for 60 minutes from 30 minutes to 90 minutes after the incubation was started, was measured using a plate reader (Varioskan Flash, Cat. No.: 5250040 manufactured by Thermo Fisher Scientific) (each group n = 3). The negative control (NC) was carried out in the same manner, except that the composition was not added. The positive control (FBS) was carried out in the same manner, except that the FBS was added such that the concentration of the FBS was 1% or 10%, instead of the composition. Further, Reference Examples (ASA, Mes) were carried out in the same manner, except that L-Ascorbic Acid (ASA) (Cat. No.: 013-19641 manufactured by Fujifilm-Wako) having collagen production promoting activity was added to have a concentration of 2 mmol/l, or that N-Methyl-L-serine (MeS) (Cat. No.: 73156 manufactured by Sigma-Aldrich) having hyaluronic acid production promoting activity was added to have a concentration of 10 mmol/l, instead of the composition. Also, the cell viability of each sample was used as a relative proliferative activity value to the cell viability in the negative control as 100%. Then, in Student's T-test (two-tailed test, unpaired) for comparison with the negative control, results with a p-value of less than 0.05 were determined as being significant. These results are shown in FIG. 4.

FIG. 4 is a graph showing the proliferative activity of fibroblasts. In FIG. 4, the type and the concentration of the samples are shown on the horizontal axis, and the cell viability (proliferative activity) is shown on the vertical axis. As shown in FIG. 4, when either the composition prepared from the platelet-removed megakaryocyte cultures (iMDF1, iMDF2) or the composition prepared from the platelets (PLT, PLTMax Human Platelet Lysate, Cat. No.: SCM141 manufactured by EMD Millipore) was added, the effect of promoting concentration-dependent cell proliferation was confirmed. Further, it was considered that the cell proliferation promoting activity of the composition of the present invention was higher than that of the FBS at the same concentration.

### (3) Confirmation of extracellular matrix production promoting activity

After the culturing for 48 hours in Example 3(2), the culture supernatant in each well was collected and stored at -80°C until the supernatant was used in Type I collagen and hyaluronic acid production promotion tests, which will be described later.

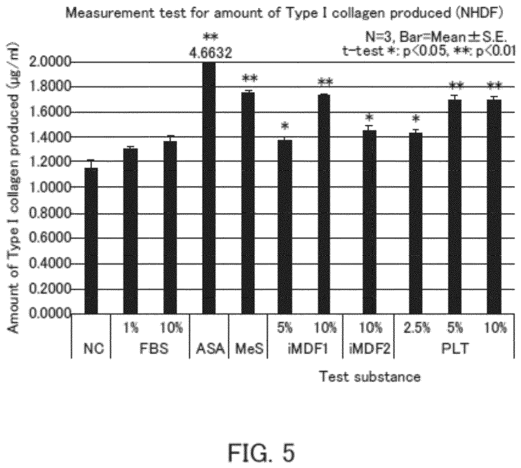

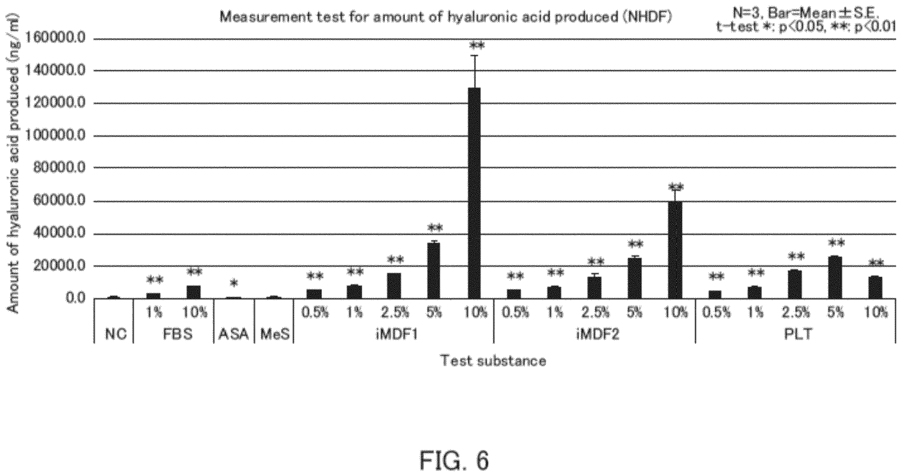

Then, the amount of Type I collagen in the culture supernatant (each group n = 3) was measured by a Type I collagen measurement kit (Human Collagen type I, ELISA kit (without pepsin), Cat. No.: EC1-E105 manufactured by ACEL). Also, the amount of hyaluronic acid in the culture supernatant was measured using a hyaluronic acid measurement kit (Hyaluronan DuoSet ELISA, Cat. No.: DY3614 manufactured by R&D Systems). The measurement methods using the kits were used according to the attached protocols. The negative control (NC) was carried out in the same manner, except that the composition was not added. With regard to the FBS added group (FBS), the measurements were carried out in the same manner as in Example 3(2). Further, the positive control (ASA or Mes) was carried out in the same manner, except that, with regard to the samples to be used in the Type I collagen measurement, L-Ascorbic Acid (ASA) (Cat. No.: 013-19641 manufactured by Fujifilm-Wako) having collagen production promoting activity was added instead of the composition so as to have a concentration of 2 mmol/l, and, with regard to the samples to be used in hyaluronic acid measurement, N-Methyl-L-serine (MeS) (Cat. No.: 73156 manufactured by Sigma-Aldrich) having hyaluronic acid production promoting activity was added instead of the composition so as to have a concentration of 10 mmol/l. Then, in Student's T-test (two-tailed test, unpaired) for comparison with the negative control, results with a p-value of less than 0.05 were determined as being significant. These results are shown in FIGS. 5 and 6.

FIG. 5 is a graph showing the amount of produced Type I collagen. In FIG. 5, the type of samples is shown on the horizontal axis, and the amount of produced Type I collagen is shown on the vertical axis. Note that the numerical value of ASA in FIG. 5 indicates the amount of collagen (µg/ml) produced in the Reference Example (ASA). As shown in FIG. 5, when either the composition prepared from the platelet-removed megakaryocyte culture or the composition prepared from the platelets was added, it was confirmed that the amount of produced Type I collagen increased concentration-dependently. Compared with the results of the cell proliferation promoting activity in Example 3(2), it was presumed that the Type I collagen production promoting activity was obtained due to cell proliferation promoting activity.

Next, FIG. 6 is a graph showing the amount of produced hyaluronic acid. In FIG. 6, the type and the concentrations of samples are shown on the horizontal axis, and the amount of produced hyaluronic acid is shown on the vertical axis. As shown in FIG. 6, when either the composition prepared from the platelet-removed megakaryocyte culture or the composition prepared from the platelets was added, it was confirmed that the amount of produced hyaluronic acid increased concentration-dependently. Compared with the cell proliferation promoting activity, it was presumed that the hyaluronic acid production promoting activity is an activity independent from the cell proliferation promoting activity, that is, the composition of the present invention acts on fibroblasts and promotes the production of hyaluronic acid.

Based on the above, it was found that the composition of the present invention has fibroblast proliferation promoting activity and the activity of promoting production of extracellular matrix components such as Type I collagen and hyaluronic acid. When skin or the like is damaged, the fibroblasts migrate to the damaged site and contribute to healing of the damaged site through cell division and secretion of extracellular matrix components. Because the composition of the present invention promotes these functions of fibroblasts, it is expected that the healing of skin disorders can be promoted.

### Example 4

It was confirmed that the composition of the present invention had keratinocyte function promoting activity.

### (1) Culturing of keratinocytes

Culturing of normal human epidermal keratinocytes (nHEK, Cat. No.: KK-4109 manufactured by KURABO) was initiated using a growth medium in a T-75 flask (manufactured by Sumilon) and a CO₂ incubator under predetermined culture conditions (under wet conditions 5% CO₂ and 37°C, and the same applies to the following). Humedia-KG2 medium (Cat. No.: KK-2150S manufactured by KURABO) was used as the growth medium. During the culturing, the culture medium was replaced every 1 to 2 days. When the cells reached about 80% confluent, the cells were collected and used in the following tests. The cells were subcultured as follows. First, after the cells was washed with phosphate buffer (PBS) (-/-) (Cat. No.: 14249-95 manufactured by Nacalai tesque), the cells were detached using a dissociation solution (2.5 g/l-Trypsin/1 mmol/l-EDTA Solution, with Phenol Red (0.25% Trypsin-EDTA), Cat. No.: 32777-44 manufactured by Thermo Fisher Scientific), and trypsin was neutralized by adding the growth medium. Then, the cell suspension was collected in a 15 ml-centrifuge tube and centrifuged (room temperature, 1000 rpm, 5 minutes) using a centrifuge (multipurpose cooled centrifuge, Cat. No.: CAX-571 manufactured by TOMY). After the centrifugation, the supernatant was removed, a new growth medium was added, cells were stirred, and the number of living cells was counted using the trypan blue assay. The cell concentration was adjusted to a desired cell concentration using the growth medium, and the cells were seeded in an incubator used in the subsequent tests.

### (2) Confirmation of keratinocyte proliferation promoting activity

The cells were seeded on a 96-well plate (Cat. No.: MS-8096F manufactured by Sumilon) at a density of 1×10³ cells/0.1 ml/well and cultured for 1 day under the above-described culture conditions. After the culturing, the culture medium was replaced with a maintenance medium (Humedia-KB2, Cat. No.: KK-2350S manufactured by KURABO) containing the composition prepared from the platelet-removed megakaryocyte culture (iMDF1, iMDF2) or the platelets (PLT) so as to achieve a predetermined concentration (each test substance treatment concentration: 0.5%, 1%, 2.5%, 5%, or 10%). Note that two types of compositions prepared from the platelet-removed megakaryocyte culture were used. After the culture medium was replaced, culturing was performed for 48 hours.

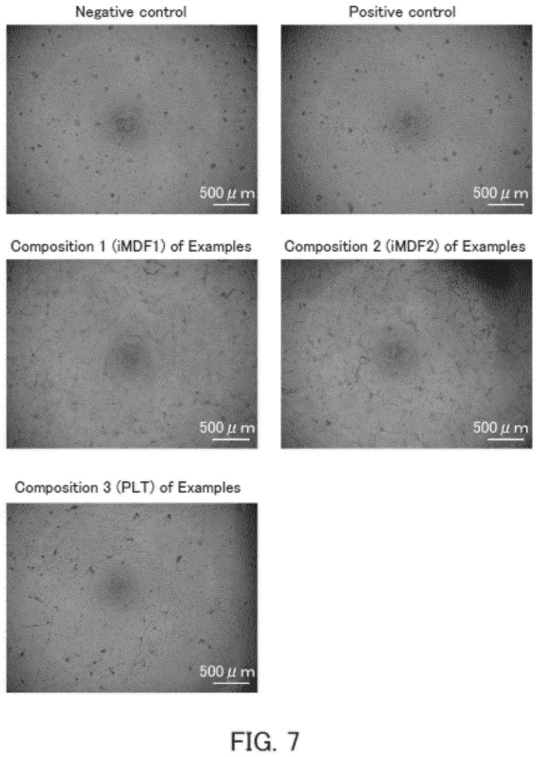

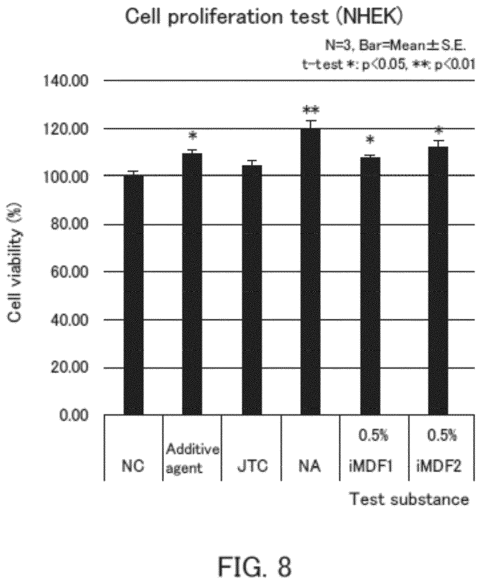

After the culturing, the morphology of cells in each well was observed using a phase-contrast microscope (Cat. No.: CKX53 manufactured by OLYMPUS). Further, with regard to the cells in each well, the amount of change in absorbance (450 nm) was measured in the same manner as in Example 3(2) above (each group n = 3). The negative control (NC) was carried out in the same manner, except that the composition was not added. The positive control (an additive agent) was carried out in the same manner, except that the growth medium was used instead of the maintenance medium containing the composition. Also, Reference Example (JTC or NA) was carried out in the same manner, except that JTC-801 (JTC, Cat. No.: J3955 manufactured by Sigma-Aldrich, final concentration was 100 nmol/l) having FLG gene expression promoting activity or Nicotinamide (NA, Cat. No.: N0636-100G manufactured by Sigma-Aldrich, final concentration was 30 µmol/l) having SPTLC1 gene expression promoting activity was added instead of the composition. Also, the cell viability of each sample was used as a relative proliferative activity value to the cell viability in the negative control as 100%. Then, in Student's T-test (two-tailed test, unpaired) for comparison with the negative control, results with a p-value of less than 0.05 were determined as being significant. These results are shown in FIGS. 7 and 8.

FIG. 7 is photographs showing phase-contrast images of cells in each well. Note that, as for the composition added groups, in which the composition was prepared from the platelet-removed megakaryocyte culture (iMDF1, iMDF2) or the platelets (PLT), an example of 10% additive group is used as a representative example. As shown in FIG. 7, in the negative control, the boundaries between keratinocytes were clear, and differentiation into cells (epidermal cells) in the epithelial (epidermal) layer was not observed. On the other hand, as for the composition prepared from the platelet-removed megakaryocyte culture (iMDF1, iMDF2) or the platelets (PLT), the keratinocytes elongated on the plate, and the cell boundaries were unclear. This is because the keratinocytes were differentiated into cells (epidermal cells) in the epithelial (epidermal) layer. Also, as for the composition prepared from the platelet-removed megakaryocyte culture (iMDF1, iMDF2) or the platelets (PLT), the ratio of the cells in the epithelial (epidermal) layer increased concentration-dependently, and in particular, when the platelet-removed megakaryocyte culture (iMDF1, iMDF2) was used, a significant increase in the cell ratio was observed. Based on these results, it was found that the composition of the present invention promotes cell differentiation of keratinocytes into the epithelial layer.

FIG. 8 is a graph showing the keratinocyte proliferative activity. In FIG. 8, the type and the concentration of samples are shown on the horizontal axis, and the cell viability (proliferative activity) is shown on the vertical axis. As shown in FIG. 8, when either the composition prepared from the platelet-removed megakaryocyte culture (iMDF1, iMDF2) or the composition prepared from the platelets (PLT) was added, the effect of promoting cell proliferation was confirmed. Further, it was considered that the cell proliferation promoting activity of the composition of the present invention was higher than that of the FBS at the same concentration.

### (3) Confirmation of activity of promoting induction of barrier function gene

Keratinocytes were cultured in the same manner as in Example 4(2), except that the keratinocytes were seeded on a 24-well plate (Cat. No.: MS-8024 manufactured by Sumilon) at a density of 5×10⁴ cells/0.5 ml/well. After the culturing, the Total RNA was collected from the cells and purified using an RNA extraction kit (RNeasy 96 Kit, Cat. No.: 74181 manufactured by QIAGEN). RNA was extracted according to the protocol attached to the kit. The obtained purified RNA was stored at -80°C until the concentration thereof was measured using a spectrophotometer (NanoDrop One, Cat. No.: ND-ONE-W manufactured by Thermo Fisher Scientific), and the purity was checked by A260/A280 and used for reverse transcription.

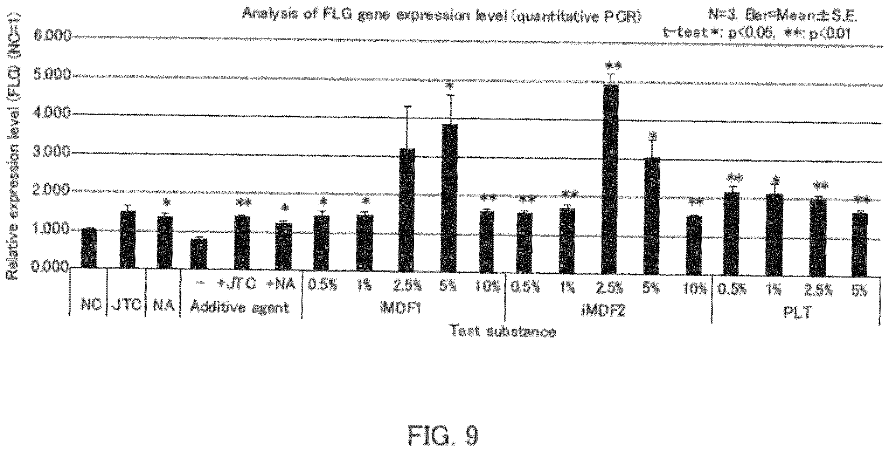

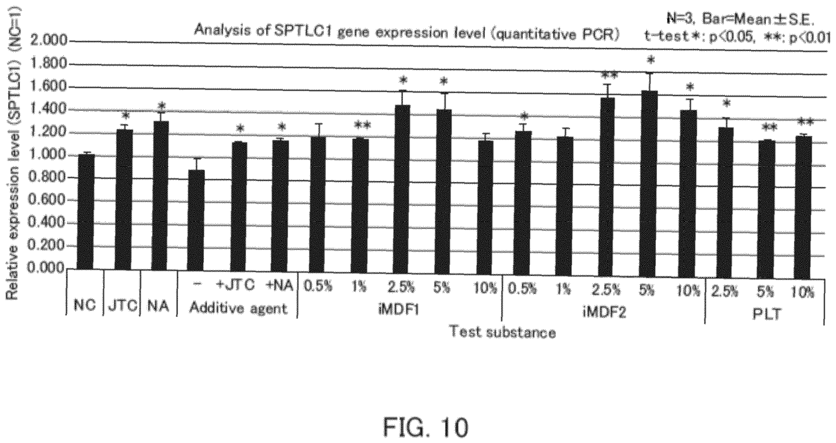

cDNA was synthesized from the purified RNA using a reverse transcription kit (QuantiTect Reverse Transcription Kit manufactured by QIAGEN). The reverse transcription was carried out according to the protocols attached to the kit. The obtained cDNA was stored at -30°C. Then, quantitative PCR was carried out using the obtained cDNA, a primer set for GAPDH gene, FLG gene, or SPTLC1 gene below, and a qPCR kit (TB Green Premix Ex Taq II (Tli RNaseH Plus), Cat. No.: RR820A manufactured by Takara). The PCR reaction was carried out by performing a heat treatment at 95°C for 30 seconds, and carrying out 40 cycles at 95°C for 5 seconds and 60°C for 30 seconds as one cycle, and the obtained PCR product was dissociated. The qPCR was carried out using a qPCR device (LightCycler 96 Instrument, Cat. No.: 05815916001 manufactured by Roche). The negative control (NC) was carried out in the same manner, except that the composition was not added. The positive control (with JTC or NA) was carried out in the same manner, except that, with regard to samples to be used in the FLG gene expression measurement, JTC-801 (JTC, Cat. No.: J3955 manufactured by Sigma-Aldrich) having FLG gene expression promoting activity was added, instead of the composition, so as to have a concentration of 100 nmol/l, and, with regard to samples to be used in SPTLC1 gene expression measurement, Nicotinamide (NA, Cat. No.: N0636-100G manufactured by Sigma-Aldrich) having SPTLC1 gene expression promoting activity was added, instead of the composition, so as to have a concentration of 30 µmol/l. Also, in the same manner, the groups to which additive agents were added were prepared, and the negative control and the positive control were carried out. The expression level of each gene corrected by the expression level of the GAPDH gene was calculated using the ΔΔCt method based on the obtained measurement data, and the relative expression levels were calculated as the expression level of the negative control was 1. The obtained values were compared with the negative control, and results with a p-value of less than 0.05 in Student's T-test (two-tailed test, unpaired) were determined as being significant. These results are shown in FIGS. 9 and 10.
- Primer set for the GAPDH gene
   Forward primer (Sequence ID No. 1)
      5'-CATCCCTGCCTCTACTGGCGCTGCC-3'
   Reverse primer (Sequence ID No. 2)
      5'-CCAGGATGCCCTTGAGGGGGCCCTC-3'
- Primer set for the FLG gene
   Forward primer (Sequence ID No. 3)
      5'-TCGGCAAATCCTGAAGAATCCAGA-3'
   Reverse primer (Sequence ID No. 4)
      5'-GCTTGAGCCAACTTGAATACCATCAG-3'
- Primer set for the SPTLC1 gene
   Forward primer (Sequence ID No. 5)
      5'-ACAAAGCAAGAATCTTCCTGGAGGAAAGCC-3'
   Reverse primer (Sequence ID No. 6)
      5'-AAACCTCCAATAGAAGCAAGTGCATTCTCC-3'

FIG. 9 is a graph showing the expression level of the FLG gene. In FIG. 9, the type and the concentrations of the samples are shown on the horizontal axis, and the expression level of the FLG gene is shown on the vertical axis. As shown in FIG. 9, when either the composition prepared from the platelet-removed megakaryocyte culture (iMDF1, iMDF2) or the composition prepared from the platelets (PLT) was added, the effect of promoting FLG gene expression was confirmed. Further, it was considered that the FLG gene expression promoting activity of the composition of the present invention was higher than that of JTC801 of the positive control at the same concentration.

Next, FIG. 10 is a graph showing the expression level of the SPTLC1 gene. In FIG. 10, the type and the concentrations of the samples are shown on the horizontal axis, and the expression level of SPTLC1 gene is shown on the vertical axis. As shown in FIG. 10, when either the composition prepared from the platelet-removed megakaryocyte culture (iMDF1, iMDF2) or the composition prepared from the platelets (PLT) was added, the effect of promoting SPTLC1 gene expression was confirmed. Further, it was considered that the SPTLC1 gene expression promoting activity of the composition of the present invention was higher than that of nicotinamide (NA) of the positive control at the same concentration.

Based on the above, it was found that the composition of the present invention has keratinocyte proliferation promoting activity, promotes differentiation into epidermal cells, and has the activity of promoting expression of barrier function genes such as the FLG gene and the SPTLC1 gene. It is known that proliferation of the keratinocytes, differentiation into epidermal cells, and induction of expression of barrier function genes are important for maintaining skin tissue and the barrier function thereof. Therefore, because the composition of the present invention promotes these functions of keratinocytes, it is expected that the composition can be used for maintaining skin conditioning such as maintenance of skin functions and maintenance of skin barrier function.

### Example 5

It was confirmed that the composition of the present invention had dermal papilla cell function promoting activity.

### (1) Culturing of dermal papilla cells

Human hair dermal papilla cells (HFDPC, Cat. No.: CA60205a manufactured by TOYOBO) were initiated using a growth medium in a T-75 flask (manufactured by Sumilon), and were cultured using a CO₂ incubator under predetermined culture conditions (under wet conditions at 5% CO₂ and 37°C, and the same being applied to the followings). The growth medium was prepared by adding attached additive agents to a dermal papilla cell growth medium (PCGM, Cat. No.: TMTPGM-250S manufactured by TOYOBO). During the culturing, the culture medium was replaced every 1 to 2 days. When the cells reached about 80% confluent, the cells were collected and used in the following tests. The cells were subcultured as follows. First, after the cells was washed with phosphate buffer (PBS) (-/-) (Cat. No.: 14249-95 manufactured by Nacalai tesque), the cells were detached using a dissociation solution (2.5 g/l-Trypsin/1 mmol/l-EDTA Solution, with Phenol Red (0.25% Trypsin-EDTA), Cat. No.: 32777-44 manufactured by Thermo Fisher Scientific), and trypsin was neutralized by adding the growth medium. Then, the cell suspension was collected in a 15 ml-centrifuge tube and centrifuged (room temperature, 1000 rpm, 5 minutes) using a centrifuge (multipurpose cooled centrifuge, Cat. No.: CAX-571 manufactured by TOMY). After the centrifugation, the supernatant was removed, a new growth medium was added, cells were stirred, and a number of living cells was counted using the trypan blue assay. The cell concentration was adjusted to a desired cell concentration using the growth medium, and the cells were seeded in an incubator used in the subsequent tests.

### (2) Confirmation of dermal papilla cell proliferation promoting activity

The cells were seeded on a 96-well plate (Cat. No.: MS-8096F manufactured by Sumilon) at a density of 5×10³ cells/0.1 ml/well and cultured for 1 day under the above-described culture conditions. After the culturing, the culture medium was replaced with the dermal papilla cell growth medium containing the composition prepared from the platelet-removed megakaryocyte culture (iMDF1, iMDF2) or the platelets (PLT) so as to achieve a predetermined concentration (each test substance treatment concentration: 0.5%, 1%, 2.5%, 5%, or 10%). Note that two types of compositions prepared from the platelet-removed megakaryocyte culture were used. After the culture medium was replaced, culturing was performed for 48 hours.

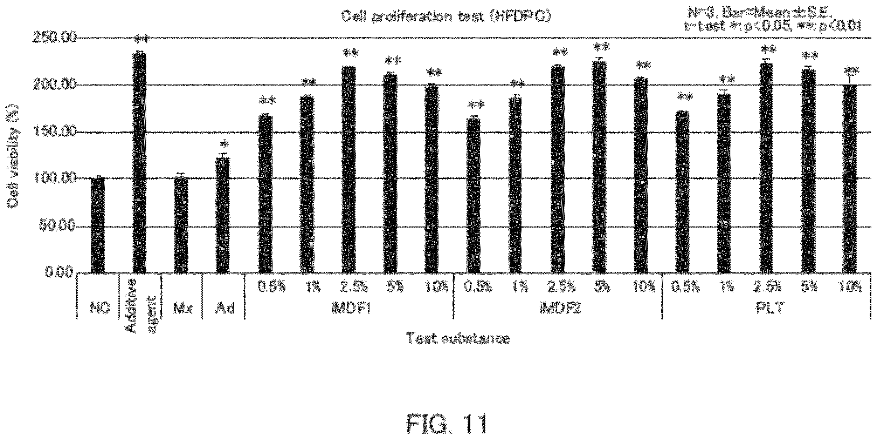

After the culturing, with regard to the cells in each well, the amount of change in absorbance (450 nm) was measured in the same manner as in Example 3(2) above (in each group, n = 3). The negative control (NC) was carried out in the same manner, except that the composition was not added. The positive control (an additive agent) was carried out in the same manner, except that the dermal papilla cell growth medium containing the additive agent was used. Reference Example (Mx or Ad) was carried out in the same manner, except that Minoxidil (Mx, Cat. No.: M4145 manufactured by Sigma-Aldrich, final concentration was 30 µmol/l) or Adenosine (Ad, Cat. No.: A9251 manufactured by Sigma-Aldrich, final concentration was 100 µmol/l), which is known as a hair growth agent, was added instead of the composition. Also, the cell viability of each sample was used as a relative proliferative activity value to the cell viability in the negative control as 100%. Then, in Student's T-test (two-tailed test, unpaired) for comparison with the negative control, results with a p-value of less than 0.05 were determined as being significant. These results are shown in FIG. 11.

FIG. 11 is a graph showing the dermal papilla cell proliferative activity. In FIG. 11, the type and the concentration of samples are shown on the horizontal axis, and the cell viability (proliferative activity) is shown on the vertical axis. As shown in FIG. 11, when either the composition prepared from the platelet-removed megakaryocyte culture (iMDF1, iMDF2) or the composition prepared from the platelets (PLT) was added, the effect of promoting cell proliferation was confirmed. Also, it was suggested that as the addition amount, 2.5% to 5% were pointed out.

### (3) Confirmation of activity of promoting induction of hair growth promoting gene

Dermal papilla cells were cultured in the same manner as in Example 5(2), except that the dermal papilla cells were seeded on a 24-well plate (Cat. No.: MS-8024 manufactured by Sumilon) at a density of 2.5×10⁴ cells/0.5 ml/well. After the culturing, cDNA was synthesized in the same manner as in Example 4(3).

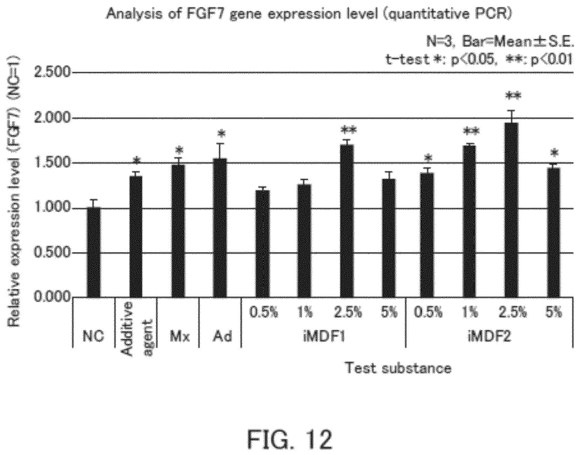

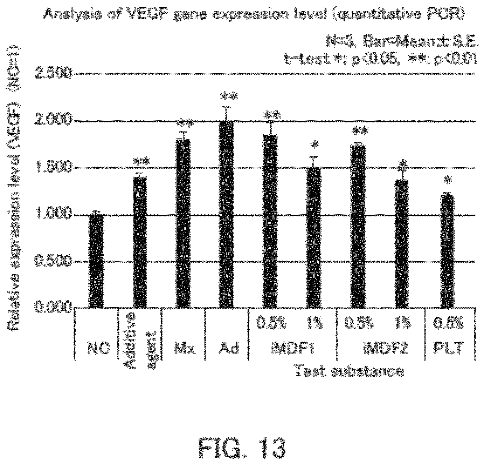

Then, qPCR was carried out in the same manner as in Example 4(3) above, except that a primer set for the above GAPDH gene, the FGF7 gene, or the VEGFA gene below was used as a primer set. The negative control was carried out in the same manner, except that the composition was not added. Reference Example was carried out in the same manner, except that Minoxidil (Cat. No.: M4145 manufactured by Sigma-Aldrich, final concentration was 30 µmol/l) or Adenosine (Cat. No.: A9251 manufactured by Sigma-Aldrich, final concentration was 100 µmol/l), which is known as a hair growth agent, was added instead of the composition. The expression level of each gene corrected by the expression level of the GAPDH gene was calculated using the ΔΔCt method based on the obtained measurement data, and relative expression level was calculated as the expression level of the negative control was 1. The obtained values were compared with the negative control, and results with a p-value of less than 0.05 in Student's T-test (two-tailed test, unpaired) were determined as being significant. These results are shown in FIGS. 12 and 13.

### • Primer set for the FGF7 gene

Forward primer (Sequence ID No. 7)
   5'-TCTGTCGAACACAGTGGTACCTGAG-3'
Reverse primer (Sequence ID No. 8)
   5'-GCCACTGTCCTGATTTCCATGA-3'

### • Primer set for the VEGFA gene

Forward primer (Sequence ID No. 9)
   5'-AAAGCATTTGTTTGTACAAGATCCG-3'
Reverse primer (Sequence ID No. 10)
   5'-CTTGTCACATCTGCAAGTACGTTCG-3'

FIG. 12 is a graph showing the expression level of FGF7 gene. In FIG. 12, the type of the samples is shown on the horizontal axis, and the expression level of FGF7 gene is shown on the vertical axis. As shown in FIG. 12, when the composition prepared from the platelet-removed megakaryocyte culture (iMDF1, iMDF2) was added, the effect of promoting FGF7 gene expression was confirmed. Further, it was considered that the FGF7 gene expression promoting activity of the composition of the present invention was almost the same as that of the known hair growth agents at the same concentration.

Next, FIG. 13 is a graph showing the expression level of the VEGFA gene. In FIG. 13, the type of samples is shown on the horizontal axis, and the expression level of VEGFA gene is shown on the vertical axis. As shown in FIG. 13, when the composition prepared from the platelet-removed megakaryocyte culture (iMDF1, iMDF2) or the composition prepared from the platelets (PLT) was added, the effect of promoting VEGFA gene expression was confirmed. Further, it was considered that the VEGFA gene expression promoting activity of the composition of the present invention was almost the same as that of the known hair growth agents at the same concentration.

Based on the above, it was found that the composition of the present invention has the activity of promoting proliferation of dermal papilla cells, and the activity of promoting expression of hair growth genes such as FGF7 gene and VEGFA gene. It is known that the proliferation of dermal papilla cells and the induction of expression of hair growth genes are important for regrowing hair, and growing and maintaining hair. Therefore, the composition of the present invention promotes these functions of dermal papilla cells, and thus, it is expected that the composition can be used for hair growth.

Although the present invention was described with reference to the embodiments and the examples above, the present invention is not limited to the above embodiments and examples. Various changes that can be understood by those skilled in the art can be made to the configuration and details of the present invention within the scope of the present invention.

This application is based upon and claims the benefit of priority from Japanese Patent Application No. 2019-225959, filed on December 13, 2019, and the entire content of the disclosure is incorporated herein by reference.

### Supplementary Descriptions

Some or all of the above embodiments and examples may be described as, but not limited to, the following supplementary descriptions.

### <Composition>

### (Supplementary Description 1)

A composition containing a treated product of megakaryocytes or a culture of the megakaryocytes.

### (Supplementary Description 2)

The composition according to Supplementary Description 1, wherein the treated product is an extract of a cell fraction of the megakaryocytes or the culture of the megakaryocytes.

### (Supplementary Description 3)

The composition according to Supplementary Description 1 or 2, wherein the treated product contains a basic fibroblast growth factor (bFGF) in an amount of 2000 to 20000 pg in 1 mg of a total protein.

### (Supplementary Description 4)

The composition according to any one of Supplementary Descriptions 1 to 3, wherein the treated product contains an insulin-like growth factor-binding protein-2 (IGFBP-2) in an amount of 8000 to 80000 pg in 1 mg of the total protein.

### (Supplementary Description 5)

The composition according to any one of Supplementary Descriptions 1 to 4, wherein the treated product contains a placental growth factor (PIGF) in an amount of 1 to 60 pg in 1 mg of the total protein.

### (Supplementary Description 6)

The composition according to any one of Supplementary Descriptions 1 to 5, wherein the treated product contains stem cell factor receptor (SCFR) in an amount of 200 to 2000 pg in 1 mg of the total protein.

### (Supplementary Description 7)

The composition according to any one of Supplementary Descriptions 1 to 6, wherein the treated product contains a vascular endothelial growth factor (VEGF) in an amount of 20 to 800 pg in 1 mg of the total protein.

### (Supplementary Description 8)

The composition according to any one of Supplementary Descriptions 1 to 7, wherein the treated product contains vascular endothelial growth factor receptor 2 (VEGFR2) in an amount of 20 to 400 pg in 1 mg of the total protein.

### (Supplementary Description 9)

The composition according to any one of Supplementary Descriptions 1 to 8, wherein the treated product contains a growth differentiation factor-15 (GDF-15) in an amount of 1000 to 10000 pg in 1 mg of the total protein.

### (Supplementary Description 10)

The composition according to any one of Supplementary Descriptions 1 to 9, wherein the treated product contains a bone morphogenetic protein-7 (BMP-7) in an amount of 0 to 1000 pg in 1 mg of the total protein.

### (Supplementary Description 11)

The composition according to any one of Supplementary Descriptions 1 to 10, wherein the treated product contains an amphiregulin (AR) in an amount of 0 to 16 pg in 1 mg of a total protein.

### (Supplementary Description 12)

The composition according to any one of Supplementary Descriptions 1 to 11, wherein the treated product contains an epidermal growth factor receptor (EGFR) in an amount of 0 to 60 pg in 1 mg of the total protein.

### (Supplementary Description 13)

The composition according to any one of Supplementary Descriptions 1 to 12, wherein the treated product contains a hepatocyte growth factor (HGF) in an amount of 0 to 100 pg in 1 mg of the total protein.

### (Supplementary Description 14)

The composition according to any one of Supplementary Descriptions 1 to 13, wherein the treated product contains an insulin-like growth factor-binding protein-1 (IGFBP-1) in an amount of 0 to 200 pg in 1 mg of the total protein.

### (Supplementary Description 15)

The composition according to any one of Supplementary Descriptions 1 to 14, wherein the composition has cell proliferation promoting activity.

### (Supplementary Description 16)

The composition according to Supplementary Description 15, wherein the cell is a mesenchymal stem cell, a fibroblast, a keratinocyte, and/or a dermal papilla cell.

### (Supplementary Description 17)

The composition according to any one of Supplementary Descriptions 1 to 16, wherein the composition has a fibroblast function promoting activity.

### (Supplementary Description 18)

The composition according to Supplementary Description 17, wherein the function of the fibroblast is proliferation of the fibroblast and/or production of an extracellular matrix by the fibroblast.

### (Supplementary Description 19)

The composition according to Supplementary Description 18, wherein the extracellular matrix contains collagen and/or hyaluronic acid.

### (Supplementary Description 20)

The composition according to any one of Supplementary Descriptions 1 to 19, wherein the composition has keratinocyte function promoting activity.

### (Supplementary Description 21)

The composition according to Supplementary Description 20, wherein the function of the keratinocyte is proliferation of the keratinocyte, differentiation into epidermal cells, and/or induction of a barrier function gene.

### (Supplementary Description 22)

The composition according to Supplementary Description 21, wherein the barrier function gene includes the profilaggrin gene and/or ceramide synthase gene.

### (Supplementary Description 23)

The composition according to any one of Supplementary Descriptions 1 to 22, wherein the composition has dermal papilla cell function promoting activity.

### (Supplementary Description 24)

The composition according to Supplementary Description 23, wherein the function of the dermal papilla cell is proliferation of the dermal papilla cell and/or induction of a hair growth promoting gene.

### (Supplementary Description 25)

The composition according to Supplementary Description 24, wherein the hair growth gene includes a FGF7 gene and/or a VEGF gene.

### (Supplementary Description 26)

The composition according to any one of Supplementary Descriptions 1 to 25, wherein the culture of the megakaryocytes is a culture from which platelets are removed.

### (Supplementary Description 27)

The composition according to any one of Supplementary Descriptions 1 to 26, wherein the megakaryocytes are immortalized megakaryocytes.

### (Supplementary Description 28)

The composition according to Supplementary Description 27, wherein the immortalized megakaryocytes are megakaryocytes that contain exogenous a BMI1 gene, a MYC gene, and a Bcl-xL gene.

### (Supplementary Description 29)

The composition according to any one of Supplementary Descriptions 1 to 28, wherein the megakaryocytes are megakaryocytes induced *in vitro.*

### (Supplementary Description 30)

The composition according to any one of Supplementary Descriptions 1 to 29, wherein the megakaryocytes are derived from pluripotent cells.

### (Supplementary Description 31)

The composition according to Supplementary Description 30,
wherein the pluripotent cells are induced pluripotent stem (iPS) cells.

### (Supplementary Description 32)

The composition according to any one of Supplementary Descriptions 1 to 31,
wherein the treated product is obtained by treating the megakaryocytes or a culture of the megakaryocytes, and
the treatment is a concentrating treatment, a drying treatment, a freezing treatment, a freeze-drying treatment, a solvent treatment, a surfactant treatment, an enzyme treatment, a protein fraction extraction treatment, a sonication treatment, and/or a disruption treatment.

### (Supplementary Description 33)

The composition according to Supplementary Description 32,
wherein the treated product is obtained by
removing platelets from the megakaryocytes or the culture of the megakaryocytes, and
treating the megakaryocytes or the culture of the megakaryocytes from which the platelets are removed.

### (Supplementary Description 34)

The composition according to Supplementary Description 33,
wherein the treated product is obtained by
storing the megakaryocytes or the culture of the megakaryocytes from which the platelets are removed, and
treating the stored megakaryocytes or the stored culture of the megakaryocytes.

### (Supplementary Description 35)

The composition according to Supplementary Description 34,
wherein the treated product is obtained by
storing the megakaryocytes or the culture of the megakaryocytes, and
subjecting the stored megakaryocytes or the stored culture of the megakaryocytes to a disruption treatment.

### <Method for producing composition>

### (Supplementary Description 36)

A method for producing a composition, including
a treating step of treating megakaryocytes or a culture of the megakaryocytes,
wherein treatment in the treating step is a concentrating treatment, a drying treatment, a freezing treatment, a freeze-drying treatment, a solvent treatment, a surfactant treatment, an enzyme treatment, a protein fraction extraction treatment, a sonication treatment, and/or a disruption treatment.

### (Supplementary Description 37)

The production method according to Supplementary Description 36, further including
a removing step of removing platelets from the megakaryocytes or the culture of the megakaryocytes,
wherein megakaryocytes or a culture of the megakaryocytes from which the platelets are removed is treated in the treating step.

### (Supplementary Description 38)

The production method according to Supplementary Description 37, further including
a storing step of storing the megakaryocytes or the culture of the megakaryocytes from which the platelets are removed,
wherein the stored megakaryocytes or the stored culture of the megakaryocytes is treated in the treating step.

### (Supplementary Description 39)

The production method according to Supplementary Description 38, further including
a storing step of storing the megakaryocytes or the culture of the megakaryocytes,
wherein, in the treating step, the stored megakaryocytes or the stored culture of the megakaryocytes is subjected to a disruption treatment.

### (Supplementary Description 40)

A composition obtained using the production method according to any one of Supplementary Descriptions 36 to 39.

### <Cell proliferation promoting composition>

### (Supplementary Description 41)

A cell proliferation promoting composition including the composition according to any one of Supplementary Descriptions 1 to 35.

### (Supplementary Description 42)

The cell proliferation promoting composition according to Supplementary Description 42, wherein the cell is a mesenchymal stem cell, a fibroblast, a keratinocyte, and/or a dermal papilla cell.

### <Method for promoting proliferation of cells>

### (Supplementary Description 43)

A method for promoting proliferation of a cell, wherein the cell proliferation promoting composition according to Supplementary Description 41 or 42 is used.

### (Supplementary Description 44)

The method for promoting proliferation of a cell according to Supplementary Description 43, wherein the cell is a mesenchymal stem cell, a fibroblast, a keratinocyte, and/or a dermal papilla cell.

### (Supplementary Description 45)

The method for promoting proliferation of a cell according to Supplementary Description 43 or 44, wherein the cell proliferation promoting composition is used *in vitro* or *in vivo.*

### <Fibroblast function promoting composition>

### (Supplementary Description 46)

A fibroblast function promoting composition including the composition according to any one of Supplementary Descriptions 1 to 35.

### (Supplementary Description 47)

The function promoting composition according to Supplementary Description 46, wherein the function of the fibroblast is proliferation of the fibroblast and/or production of an extracellular matrix by the fibroblast.

### (Supplementary Description 48)

The function promoting composition according to Supplementary Description 47, wherein the extracellular matrix contains collagen and/or hyaluronic acid.

### <Fibroblast function promoting method>

### (Supplementary Description 49)

A fibroblast function promoting method, wherein the fibroblast function promoting composition according to any one of Supplementary Descriptions 46 to 48 is used.

### (Supplementary Description 50)

The fibroblast function promoting method according to Supplementary Description 50, wherein the fibroblast function promoting composition is used *in vitro* or *in vivo.*

### <Composition for promoting healing of skin disorders>

### (Supplementary Description 51)

A composition for promoting healing of a skin disorder, including the composition according to any one of Supplementary Descriptions 1 to 35.

### (Supplementary Description 52)

The healing promoting composition according to Supplementary Description 51, wherein the skin disorder is a skin ulcer, a pressure sore, a burn, a scar, and/or a wound.

### <Method for promoting healing of skin disorders>

### (Supplementary Description 53)

A method for promoting healing of a skin disorder, wherein the composition for promoting healing of a skin disorder according to Supplementary Description 51 or 52 is used.

### (Supplementary Description 54)

The method for promoting healing of a skin disorder according to Supplementary Description 53, wherein the composition for promoting healing of a skin disorder is used *in vitro* or *in vivo.*

### <Keratinocyte function promoting composition>

### (Supplementary Description 55)

A keratinocyte function promoting composition including the composition according to any one of Supplementary Descriptions 1 to 35.

### (Supplementary Description 56)

The function promoting composition according to Supplementary Description 55, wherein the function of the keratinocyte is proliferation of the keratinocyte, differentiation into epidermal cells, and/or induction of a barrier function gene.

### (Supplementary Description 57)

The function promoting composition according to Supplementary Description 56, wherein the barrier function gene includes the profilaggrin gene and/or ceramide synthase gene.

### <Keratinocyte function promoting method>

### (Supplementary Description 58)

A keratinocyte function promoting method, wherein the keratinocyte function promoting composition according to any one of Supplementary Descriptions 55 to 57 is used.

### (Supplementary Description 59)

The keratinocyte function promoting method according to Supplementary Description 58, wherein the keratinocyte function promoting composition is used *in vitro* or *in vivo.*

### Dermal papilla cell function promoting composition

### (Supplementary Description 60)

A dermal papilla cell function promoting composition including the composition according to any one of Supplementary Descriptions 1 to 35.

### (Supplementary Description 61)

The function promoting composition according to Supplementary Description 56, wherein the function of the dermal papilla cell is proliferation of the dermal papilla cell and/or induction of a hair growth promoting gene.

### (Supplementary Description 62)

The function promoting composition according to Supplementary Description 57, wherein the barrier function gene includes the profilaggrin gene and/or ceramide synthase gene.

### <Dermal papilla cell function promoting method>

### (Supplementary Description 63)

A dermal papilla cell function promoting method, wherein the dermal papilla cell function promoting composition according to any one of Supplementary Descriptions 60 to 62 is used.

### (Supplementary Description 64)

The dermal papilla cell function promoting method according to Supplementary Description 63, wherein the dermal papilla cell function promoting composition is used *in vitro* or in vivo.

### <Hair growth promoting composition>

### (Supplementary Description 65)

A hair growth promoting composition including the composition according to any one of Supplementary Descriptions 1 to 35.

### <Hair growth promoting method>

### (Supplementary Description 66)

A hair growth promoting method, wherein the hair growth promoting composition according to Supplementary Description 65 is used.

### (Supplementary Description 67)

The hair growth promoting method according to Supplementary Description 66, wherein the hair growth promoting composition is used *in vitro* or *in vivo.*

### <Use of composition>

### (Supplementary Description 68)

A composition for use for promoting cell proliferation, including, as an active ingredient, a treated product of megakaryocytes or a culture of the megakaryocytes.

### (Supplementary Description 69)

A composition for use for promoting the function of fibroblasts, including, as an active ingredient, a treated product of megakaryocytes or a culture of the megakaryocytes.

### (Supplementary Description 70)

A composition for use for promoting healing of a skin disorder, including, as an active ingredient, a treated product of megakaryocytes or a culture of the megakaryocytes.

### (Supplementary Description 71)

A composition for use for promoting the function of keratinocytes, including, as an active ingredient, a treated product of megakaryocytes or a culture of the megakaryocytes.

### (Supplementary Description 72)

A composition for use for promoting the function of dermal papilla cells, including, as an active ingredient, a treated product of megakaryocytes or a culture of the megakaryocytes.

### (Supplementary Description 73)

A composition for use for promoting hair growth, including, as an active ingredient, a treated product of megakaryocytes or a culture of the megakaryocytes.

### Industrial Applicability

As described above, according to the present invention, it is possible to provide a composition having cell-derived physiological activity. Further, it is expected that it is possible to suitably use the composition of the present invention for promoting proliferation of cells, such as mesenchymal cells, fibroblasts, keratinocytes, and dermal papilla cells, for example, for promoting healing of skin disorders such as skin ulcers, pressure sores, burns, scars, and wounds, for maintaining or improving the barrier function of skin, and for hair growth and the like. Therefore, the present invention is extremely useful in the fields of medicine, pharmacy, regenerative medicine, and the like.

## Claims

1. A composition comprising a treated product of megakaryocytes or a culture of the megakaryocytes.

2. The composition according to claim 1, wherein the treated product is an extract of a cell fraction of the megakaryocytes or the culture of the megakaryocytes.

3. The composition according to claim 1 or 2, wherein the treated product comprises a basic fibroblast growth factor (bFGF) in an amount of 2000 to 20000 pg in 1 mg of a total protein.

4. The composition according to any one of claims 1 to 3, wherein the treated product comprises an insulin-like growth factor-binding protein-2 (IGFBP-2) in an amount of 8000 to 80000 pg in 1 mg of a total protein.

5. The composition according to any one of claims 1 to 4, wherein the treated product comprises a placental growth factor (PIGF) in an amount of 1 to 60 pg in 1 mg of a total protein.

6. The composition according to any one of claims 1 to 5, wherein the treated product comprises a stem cell factor receptor (SCFR) in an amount of 200 to 2000 pg in 1 mg of a total protein.

7. The composition according to any one of claims 1 to 6, wherein the treated product comprises a vascular endothelial growth factor (VEGF) in an amount of 20 to 800 pg in 1 mg of a total protein.

8. The composition according to any one of claims 1 to 7, wherein the treated product comprises a vascular endothelial growth factor receptor 2 (VEGFR2) in an amount of 20 to 400 pg in 1 mg of a total protein.

9. The composition according to any one of claims 1 to 8, wherein the treated product comprises a growth differentiation factor-15 (GDF-15) in an amount of 1000 to 10000 pg in 1 mg of a total protein.

10. The composition according to any one of claims 1 to 9, wherein the treated product comprises an amphiregulin (AR) in an amount of 0 to 16 pg in 1 mg of a total protein.

11. The composition according to any one of claims 1 to 10, wherein the treated product comprises an epidermal growth factor receptor (EGFR) in an amount of 0 to 60 pg in 1 mg of a total protein.

12. The composition according to any one of claims 1 to 11, wherein the treated product comprises a hepatocyte growth factor (HGF) in an amount of 0 to 100 pg in 1 mg of a total protein.

13. The composition according to any one of claims 1 to 12, wherein the treated product comprises an insulin-like growth factor-binding protein-1 (IGFBP-1) in an amount of 0 to 200 pg in 1 mg of a total protein.

14. The composition according to any one of claims 1 to 13, wherein the composition has cell proliferation promoting activity.

15. The composition according to claim 14, wherein the cell is a mesenchymal stem cell, a fibroblast, a keratinocyte, and/or a dermal papilla cell.

16. The composition according to any one of claims 1 to 15, wherein the composition has a fibroblast function promoting activity.

17. The composition according to claim 16, wherein a function of a fibroblast is proliferation of the fibroblast and/or production of an extracellular matrix by the fibroblast.

18. The composition according to any one of claims 1 to 17, wherein the composition has keratinocyte function promoting activity.

19. The composition according to claim 18, wherein the function of the keratinocyte is proliferation of the keratinocyte, differentiation into epidermal cells, and/or induction of a barrier function gene.

20. The composition according to any one of claims 1 to 19, wherein the composition has dermal papilla cell function promoting activity.

21. The composition according to claim 20, wherein the function of the dermal papilla cell is proliferation of the dermal papilla cell and/or induction of a hair growth promoting gene.

22. The composition according to any one of claims 1 to 21, wherein the megakaryocytes are immortalized megakaryocytes.

23. The composition according to any one of claims 1 to 22, wherein the megakaryocytes are megakaryocytes induced *in vitro.*

24. A cell proliferation promoting composition comprising the composition according to any one of claims 1 to 23.

25. The cell proliferation promoting composition according to claim 24, wherein the cell is a mesenchymal stem cell.

26. A fibroblast function promoting composition comprising the composition according to any one of claims 1 to 23.

27. The function promoting composition according to claim 26, wherein the function of the fibroblast is proliferation of the fibroblast and/or production of an extracellular matrix by the fibroblast.

28. A composition for promoting healing of a skin disorder, comprising the composition according to any one of claims 1 to 23.

29. The healing promoting composition according to claim 28, wherein the skin disorder is a skin ulcer, a pressure sore, a burn, a scar, and/or a wound.

30. A keratinocyte function promoting composition comprising the composition according to any one of claims 1 to 23.

31. The function promoting composition according to claim 30, wherein the function of the keratinocyte is proliferation of the keratinocyte, differentiation into epidermal cells, and/or induction of a barrier function gene.

32. A dermal papilla cell function promoting composition comprising the composition according to any one of claims 1 to 23.

33. The function promoting composition according to claim 32, wherein the function of the dermal papilla cell is proliferation of the dermal papilla cell and/or induction of a hair growth promoting gene.

34. A hair growth promoting composition comprising the composition according to any one of claims 1 to 23.
